(19) Europäisches Patentamt / European Patent Office / Office européen des brevets

(11) EP 3 280 716 B1

(12) # EUROPÄISCHE PATENTSCHRIFT

(45) Veröffentlichungstag und Bekanntmachung des Hinweises auf die Patenterteilung:
**12.02.2020 Patentblatt 2020/07**

(21) Anmeldenummer: **16714418.7**

(22) Anmeldetag: **05.04.2016**

(51) Int Cl.:
***C07D 471/04*** $^{(2006.01)}$ ***C07D 519/00*** $^{(2006.01)}$
***A01N 43/90*** $^{(2006.01)}$

(86) Internationale Anmeldenummer:
**PCT/EP2016/057389**

(87) Internationale Veröffentlichungsnummer:
**WO 2016/162318 (13.10.2016 Gazette 2016/41)**

(54) **IMIDAZO[1,2-A]PYRIDIN-2-YL-DERIVATE ALS SCHÄDLINGSBEKÄMPFUNGSMITTEL UND DEREN ZWISCHENPRODUKTE**

IMIDAZO[1,2-A]PYRIDIN-2-YL DERIVATIVES AS PESTICIDES AND INTERMEDIATES THEREOF

DÉRIVÉS D'IMIDAZO[1,2-A]PYRIDIN-2-YL EN TANT QUE PESTICIDES ET LEURS INTERMÉDIAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2015 EP 15162775**

(43) Veröffentlichungstag der Anmeldung:
**14.02.2018 Patentblatt 2018/07**

(73) Patentinhaber: **Bayer CropScience AG**
**40789 Monheim am Rhein (DE)**

(72) Erfinder:
- **FISCHER, Rüdiger**
  **50259 Pulheim (DE)**
- **WILCKE, David**
  **40219 Düsseldorf (DE)**
- **HAGER, Dominik**
  **40789 Monheim (DE)**
- **ILG, Kerstin**
  **50670 Köln (DE)**
- **EILMUS, Sascha**
  **42799 Leichlingen (DE)**
- **GÖRGENS, Ulrich**
  **40882 Ratingen (DE)**
- **TURBERG, Andreas**
  **42781 Haan (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**EP-A1- 0 052 016** **EP-A1- 0 120 589**
**WO-A1-97/11075** **WO-A1-2011/073316**
**WO-A1-2013/018371** **WO-A1-2013/191113**
**WO-A1-2015/000715** **WO-A2-2009/023179**
**WO-A2-2011/041713**

- **XIAO-MING JI ET AL: "Direct Sulfenylation of Imidazoheterocycles with Disulfides in an Iodine-Hydrogen Peroxide System", SYNTHESIS, Bd. 47, Nr. 05, 17. Februar 2015 (2015-02-17), Seiten 659-671, XP055193243, ISSN: 0039-7881, DOI: 10.1055/s-0034-1379941**
- **YAHYA GUZEL ET AL: "Investigation of Antiulcer Activities of Imidazo[1,2-6alpha;]pyridinyl-2-alkylamino benzoxazoles and 5,6,7,8-Tetahydroimidazo[1,2-alpha;]pyridi nylbenzoxazoles with Electron-Topological (ET) Method", ARCHIV DER PHARMAZIE, Bd. 335, Nr. 1, 1. Januar 2002 (2002-01-01), Seiten 27-32, XP055271943, Weinheim ISSN: 0365-6233, DOI: 10.1002/1521-4184(200201)335:1<27::AID-ARD P27>3.0.CO;2-V**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- KATSURA Y ET AL: "STUDIES ON ANTIULCER DRUGS. I. SYNTHESIS AND ANTIULCER ACTIVITIES OF IMIDAZOÄ1,2-ALPHAÜPYRIDINYL-2-OXOBENZ OXAZO LIDINES-3-OXO-2H-1,4-BENZOXAZINES AND RELATED COMPOUNDS", CHEMICAL AND PHARMACEUTICAL BULLETIN, Bd. 39, Nr. 11, 1. November 1991 (1991-11-01), Seiten 2937-2943, XP008056522, PHARMACEUTICAL SOCIETY OF JAPAN, JP ISSN: 0009-2363
- ELLI S. HAND ET AL: "Imidazo[1,2-a]pyridines - novel substitution reactions", THE JOURNAL OF ORGANIC CHEMISTRY, Bd. 41, Nr. 22, 1. Oktober 1976 (1976-10-01), Seiten 3549-3556, XP055272058, US ISSN: 0022-3263, DOI: 10.1021/jo00884a013
- PATIL SHASHIKANT M ET AL: "DMSO-POCl3: a reagent for methylthiolation of imidazo[1,2-a]pyridines and other imidazo-fused heterocy", TETRAHEDRON, Bd. 69, Nr. 38, 12. Juli 2013 (2013-07-12) , Seiten 8255-8262, XP028688559, ISSN: 0040-4020, DOI: 10.1016/J.TET.2013.07.017
- GRASSY G ET AL: "Inhibitory effects on platelet aggregation and cyclic AMP phosphodiesterase of azaindolizine-type compounds", CHEMOMETRICS AND INTELLIGENT LABORATORY SYSTEMS, Bd. 20, Nr. 1, 1. August 1993 (1993-08-01) , Seiten 71-84, XP026502520, ELSEVIER SCIENCE PUBLISHERS B.V. AMSTERDAM, NL ISSN: 0169-7439, DOI: 10.1016/0169-7439(93)80022-A [gefunden am 1993-08-01]
- MOHAN, DARAPANENI CHANDRA ET AL: "Copper(I) Iodide Catalyzed Aerobic Oxidative C-N and C-S bond formations through C-H Activation: Synthesis of Functionalized Imidazo[1,2-a]pyridines", ASIAN JOURNAL OF ORGANIC CHEMISTRY, Bd. 3, Nr. 5, 11. März 2014 (2014-03-11), Seiten 609-613, XP002757603, ISSN: 2193-5807, DOI: 10.1002/AJOC.201402004
- TEULADE, JEAN CLAUDE ET AL: "Nucleophilic aromatic substitution reaction of some 3-nitroimidazo[1,2-a]pyridines with thioglycolate anion in DMF", JOURNAL OF ORGANIC CHEMISTRY, Bd. 46, Nr. 5, Februar 1981 (1981-02), Seiten 1026-1030, XP002757604, ISSN: 0022-3263, DOI: 10.1021/JO00318A040
- DATABASE CA [Online] CHEMICAL ABSTRACTS SERVICE, COLUMBUS, OHIO, US; ISHIDA, YASUO ET AL: "Studies on sulfonylureas with fused heterocycles. Part 1. Synthesis of novel herbicidal sulfonylurea compounds with an imidazo[1,2-a]pyridine moiety", XP002757605, gefunden im STN Database accession no. 1994:435434

## Beschreibung

Die vorliegende Erfindung betrifft neue kondensierte bicyclische Heterocyclen-Derivate der Formel (I), deren Anwendung als Akarizide und/oder Insektizide zur Bekämpfung tierischer Schädlinge, vor allem von Arthropoden und insbesondere von Insekten und Spinnentieren und Verfahren und Zwischenprodukte zu ihrer Herstellung.

Kondensierte bicyclische Heterocyclen-Derivate mit insektiziden Eigenschaften sind in der Literatur bereits beschrieben, z.B. in WO 2010/125985, WO 2012/074135, WO 2012/086848, WO 2013/018928, WO 2013/191113, WO 2014/142292, WO 2014/148451, WO 2015/000715, EP 15191440.5, EP 15197267.6 und EP 16152384.0, sowie Synthesis, Bd. 47, Nr. 05, Seiten 659-671, WO 2016/129684, WO 2017/061497.

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue kondensierte bicyclische Heterocyclen-Derivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die kondensierten bicyclischen Heterocyclen-Derivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Verbindungen der Formel (I)

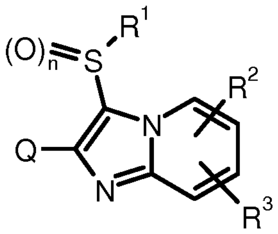
(I)

wobei $R^3$ für Wasserstoff steht und wobei $R^1$, $R^2$, Q und n, die in der folgenden Tabelle angegebenen Bedeutungen haben und wobei die Bindung von Q zum Restmolekül mit einer Wellenlinie gekennzeichnet ist:

| | $R^1$ | n | $R^2$ | Q |
|---|---|---|---|---|
| I-1 | $C_2H_5$ | 2 | H | 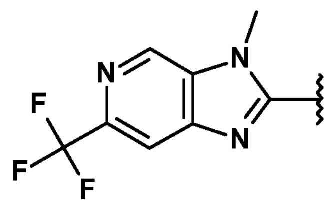 |
| I-2 | $C_2H_5$ | 0 | H | 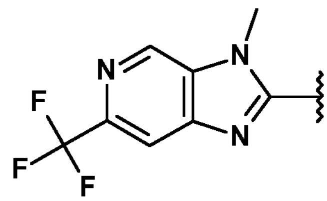 |
| I-7 | $C_2H_5$ | 1 | 6-Cl | 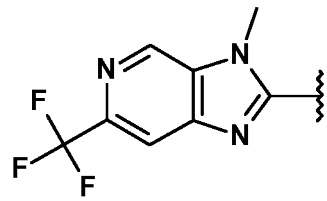 |

(fortgesetzt)

| | $R^1$ | n | $R^2$ | Q |
|---|---|---|---|---|
| I-8 | $C_2H_5$ | 1 | 6-$CF_3$ | 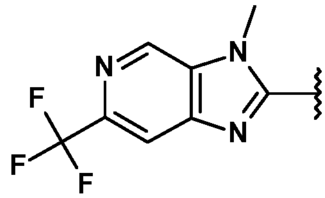 |
| I-9 | $C_2H_5$ | 0 | H | 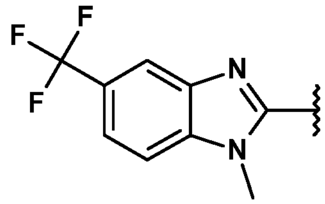 |
| I-10 | $C_2H_5$ | 2 | H | 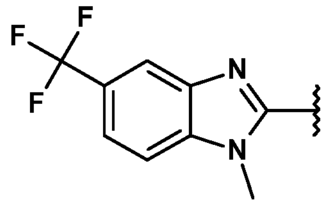 |
| I-11 | $C_2H_5$ | 1 | H | 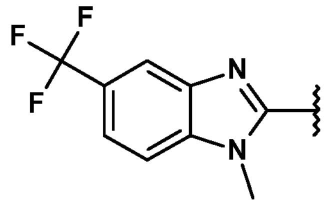 |
| I-13 | $C_2H_5$ | 0 | 8-$CF_3$ | 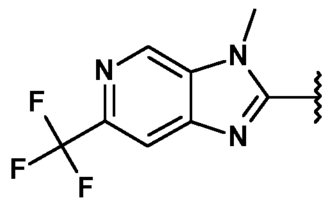 |
| I-14 | $C_2H_5$ | 0 | 8-Cl | 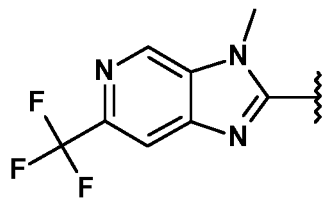 |
| I-15 | $C_2H_5$ | 0 | 8-$SC_2H_5$ | 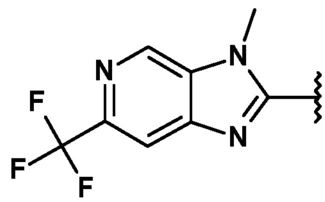 |

(fortgesetzt)

| | $R^1$ | n | $R^2$ | Q |
|---|---|---|---|---|
| I-18 | $C_2H_5$ | 1 | 7-$CF_3$ | 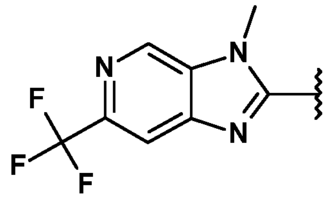 |
| I-19 | $C_2H_5$ | 2 | 8-Cl | 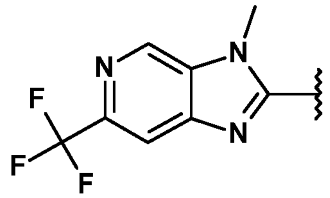 |
| I-20 | $C_2H_5$ | 0 | 5-$CF_3$ | 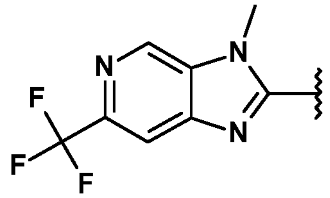 |
| I-21 | $C_2H_5$ | 2 | 5-$CF_3$ | 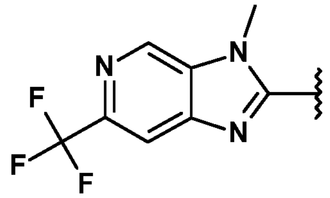 |
| I-22 | $C_2H_5$ | 1 | 5-$CF_3$ | 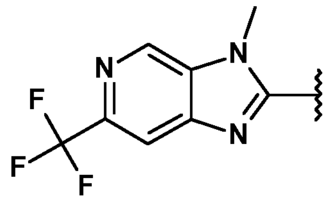 |
| I-24 | $C_2H_5$ | 2 | 8-$CF_3$ | 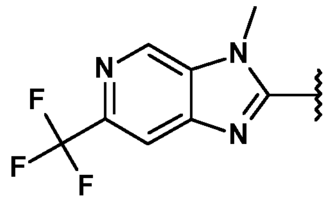 |
| I-25 | $C_2H_5$ | 1 | 8-$CF_3$ | 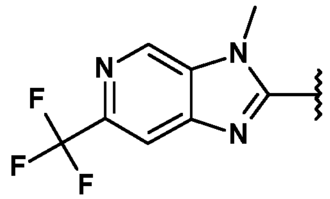 |

(fortgesetzt)

| | $R^1$ | n | $R^2$ | Q |
|---|---|---|---|---|
| I-26 | $C_2H_5$ | 0 | 5-$CH_3$ | 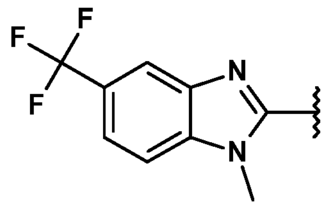 |
| I-27 | $C_2H_5$ | 0 | 5-$CF_3$ | 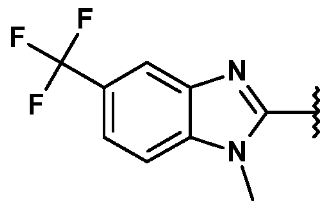 |
| I-28 | $C_2H_5$ | 0 | 6-$CF_3$ | 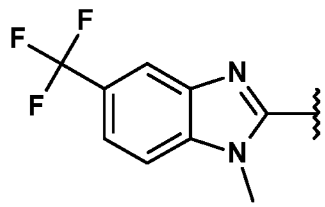 |
| I-29 | $C_2H_5$ | 0 | 6-Cl | 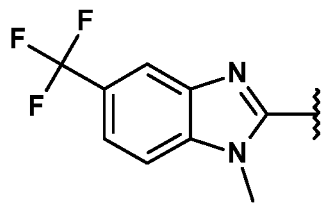 |
| I-30 | $C_2H_5$ | 0 | 7-$CF_3$ | 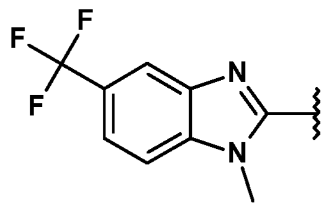 |
| I-31 | $C_2H_5$ | 0 | 5-$SC_2H_5$ | 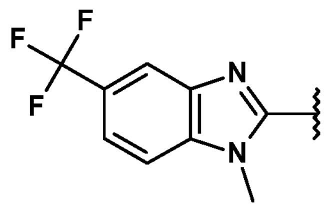 |
| I-32 | $C_2H_5$ | 0 | 7-$SC_2H_5$ | 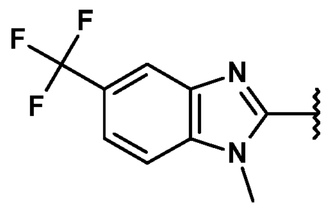 |

(fortgesetzt)

| | $R^1$ | n | $R^2$ | Q |
|---|---|---|---|---|
| I-33 | $C_2H_5$ | 2 | 7-$CH_3$ | 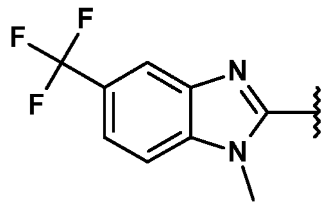 |
| I-34 | $C_2H_5$ | 2 | 7-$CF_3$ | 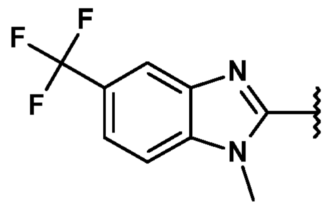 |
| I-35 | $C_2H_5$ | 2 | 6-$CH_3$ | 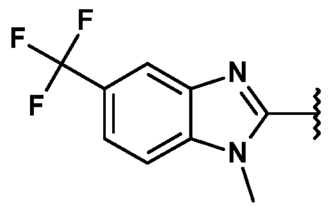 |
| I-36 | $C_2H_5$ | 2 | 6-$CF_3$ | 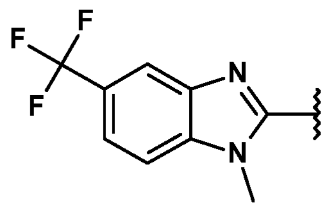 |
| I-37 | $C_2H_5$ | 0 | 7-$CH_3$ | 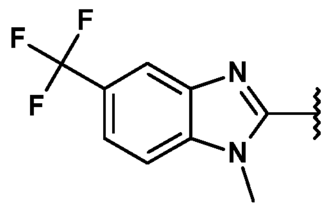 |
| I-38 | $C_2H_5$ | 0 | 6-$CH_3$ | 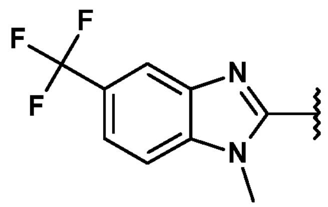 |
| I-39 | $C_2H_5$ | 2 | 8-$SO_2C_2H_5$ | 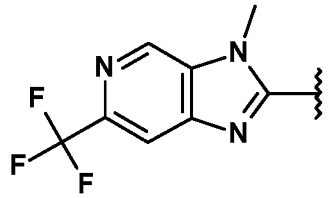 |

(fortgesetzt)

| | $R^1$ | n | $R^2$ | Q |
|---|---|---|---|---|
| I-40 | $C_2H_5$ | 2 | H | 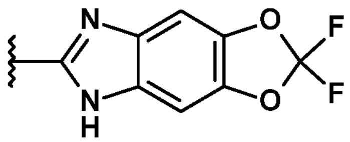 |
| I-41 | $C_2H_5$ | 2 | H | 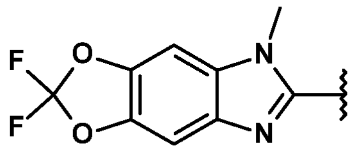 |
| I-42 | $C_2H_5$ | 2 | 6-Cl | 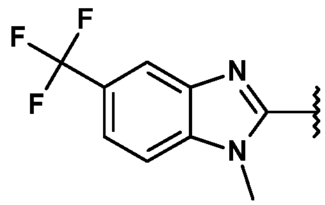 |
| I-45 | $C_2H_5$ | 0 | 6-$CH_3$ | 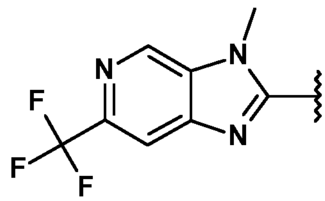 |
| I-46 | $C_2H_5$ | 2 | 5-$CH_3$ | 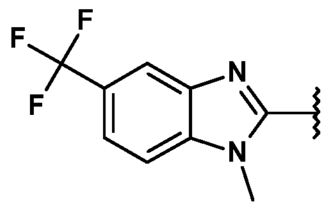 |
| I-48 | $C_2H_5$ | 2 | 6-$CH_3$ | 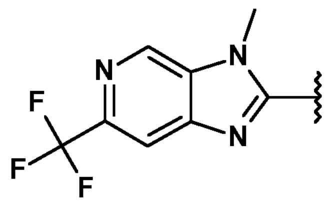 |
| I-54 | $C_2H_5$ | 2 | 6-$OCH_3$ | 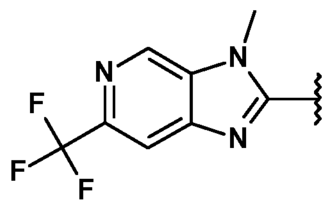 |
| I-55 | $C_2H_5$ | 1 | 6-$OCH_3$ | 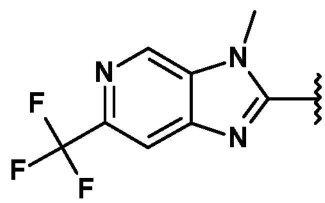 |

Weiterhin wurde gefunden, dass die Verbindungen der Formel (I) eine sehr gute Wirksamkeit als Schädlingsbekämpfungsmittel, vorzugsweise als Insektizide und/oder Akarizide aufweisen, darüber hinaus in der Regel insbesondere gegenüber Kulturpflanzen sehr gut pflanzenverträglich sind.

In den bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

In den besonders bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für $C_1$-$C_{12}$-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind $C_1$-$C_6$-Alkylreste besonders bevorzugt. Insbesondere bevorzugt sind $C_1$-$C_4$-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter $C_2$-$C_{12}$-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind $C_2$-$C_6$-Alkenylreste und besonders bevorzugt sind $C_2$-$C_4$-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter $C_2$-$C_{12}$-Alkinylrest, welcher mindestens eine Dreifachbindung aufweist, beispielsweise Ethinyl, 1-Propinyl und Propargyl, verstanden. Bevorzugt hiervon sind $C_3$-$C_6$-Alkinylreste und besonders bevorzugt sind $C_3$-$C_4$-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein $C_3$-$C_8$-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind $C_3$-$C_6$-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen der Formel (I) können durch die in den folgenden Schemata dargestellten Verfahren erhalten werden:

## Verfahren A

Die Verbindungen der Formel (I), bei denen Q für Q1 bis Q9 oder Q20 steht, können nach bekannten Methoden hergestellt werden, beispielsweise analog der in WO2009/131237, WO2010/125985, WO2011/043404, WO2011/040629, WO2012/086848, WO2013/018928 oder WO2015/000715 beschriebenen Verfahren.

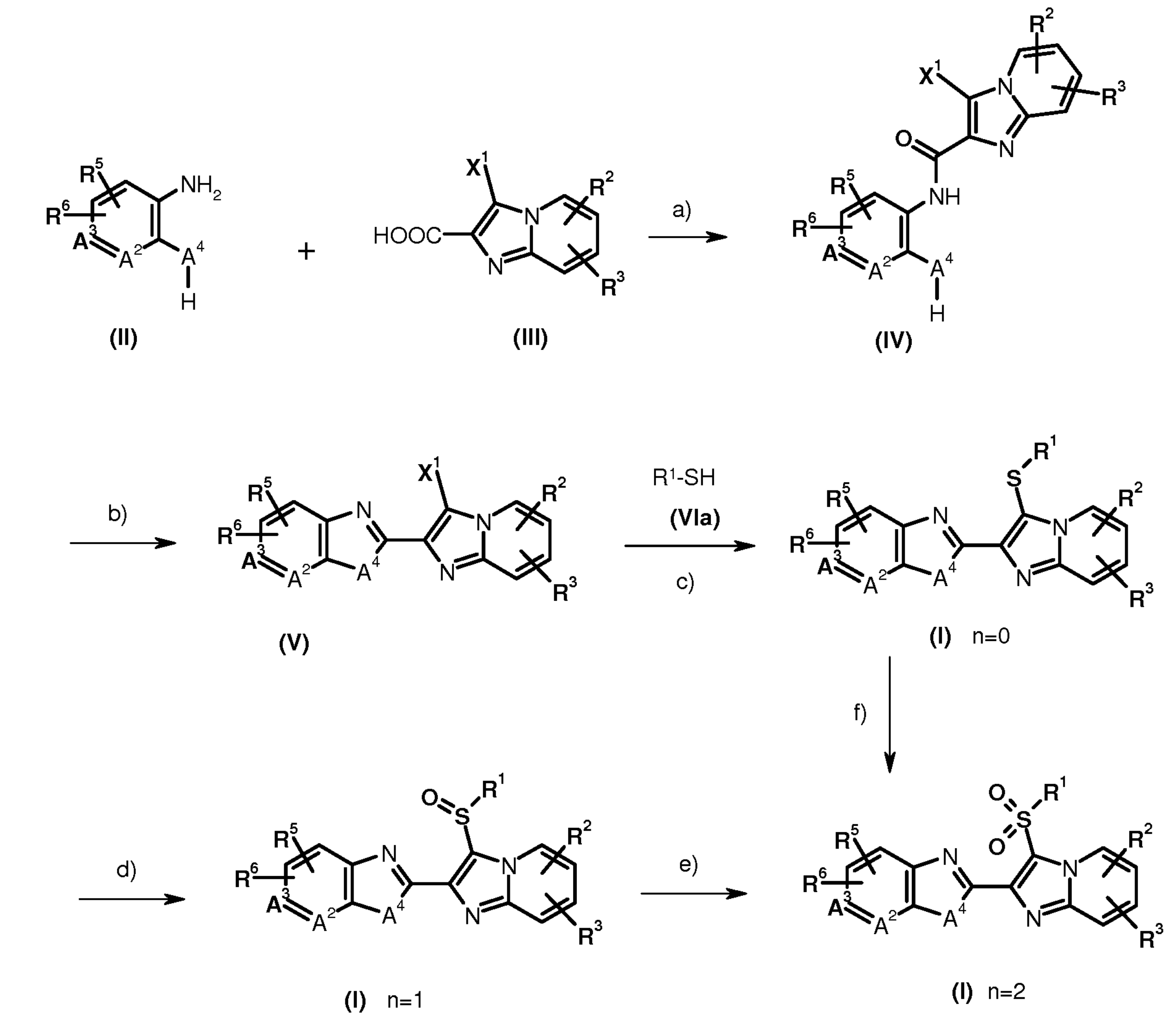


(II) (III) (IV)

(V) (I) n=0

(I) n=1 (I) n=2

Die Reste $R^1$, $R^2$, $R^3$, $R^4$, $R^5$, $R^6$ und n haben die oben beschriebenen Bedeutungen, $A^2$ und $A^3$ stehen für CH oder N (wobei $A^2$ und $A^3$ nicht gleichzeitig für N stehen können), $A^4$ steht für O, S oder N-$R^4$ und $X^1$ steht für Halogen.

**Schritt a)**

Die Verbindungen der Formel (IV) können in Analogie zu dem in US5576335 beschriebenen Verfahren durch die Umsetzung von Verbindungen der Formel (II) mit Carbonsäuren der Formel (III) in Gegenwart eines Kondensationsmittels bzw. einer Base hergestellt werden.

Verbindungen der Formel (II) sind entweder kommerziell erhältlich oder können nach bekannten Methoden hergestellt werden, beispielsweise analog der in US2003/69257, WO2006/65703, WO2009/131237, WO2010/125985, WO2011/043404, WO2011/040629, WO2012/086848, WO2013/018928 oder WO2015/000715 beschriebenen Verfahren.

Carbonsäuren der Formel (III) sind entweder kommerziell erhältlich oder können nach bekannten Methoden hergestellt werden, beispielsweise aus 2-Aminopyridin Derivaten analog der in WO2011/41713 beschriebenen Verfahren.

Die Umsetzung der Verbindungen der Formel (II) mit Carbonsäuren der Formel (III) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI) oder 1,3-Dicyclohexylcarbodiimid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

**Schritt b)**

Die Verbindungen der Formel (V) lassen sich herstellen durch Kondensation der Verbindungen der Formel (IV) z.B. analog der in WO2009/131237, WO2010/125985, WO2011/043404, WO2011/040629, WO2012/086848, WO2013/018928 oder WO2015/000715 beschriebenen Verfahren.

Die Umsetzung zu Verbindungen der Formel (V) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

**Schritt c)**

Die Verbindungen der Formel (I), wobei n für 0 steht, lassen sich herstellen durch Umsetzung der Verbindungen der Formel (V) mit den Verbindungen der Formel (VIa) in Gegenwart einer Base.

Mercaptanderivate der Formel (VIa) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindung der Formel (I), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

In der beschriebenen Reaktion steht $X^1$ bevorzugt für ein Fluor- oder Chloratom.

**Schritt d)**

Die Verbindungen der Formel (I), wobei n für 1 steht, lassen sich herstellen durch Oxidation der Verbindungen der Formel (I), wobei n für 0 steht. Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser.
Beispiele für geeignete Oxidationsmittel sind Wasserstoffperoxid, meta-Chlorperbenzoesäure oder Natriumperiodat.
Die Reaktion kann im Vakuum, bei Normaldruck oder unter Überdruck und bei Temperaturen von -20°C °C bis 120 °C durchgeführt werden.

**Schritt e)**

Die Verbindungen der Formel (I), wobei n für 2 steht, lassen sich herstellen durch Oxidation der Verbindungen der Formel (I), wobei n für 1 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser.
Beispiele für geeignete Oxidationsmittel sind Wasserstoffperoxid und meta-Chlorperbenzoesäure.
Die Reaktion kann im Vakuum, bei Normaldruck oder unter Überdruck und bei Temperaturen von -20°C °C bis 120 °C durchgeführt werden.

**Schritt f)**

Die Verbindungen der Formel (I), wobei n für 2 steht, lassen sich auch in einem einstufigen Prozess herstellen durch Oxidation der Verbindungen der Formel (I), wobei n für 0 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser.
Beispiele für geeignete Oxidationsmittel sind Wasserstoffperoxid und meta-Chlorperbenzoesäure.
Die Reaktion kann im Vakuum, bei Normaldruck oder unter Überdruck und bei Temperaturen von -20°C °C bis 120 °C durchgeführt werden.

**Verfahren B**

Die Verbindungen der Formel (I), bei denen Q für Q10, Q11, Q15 und Q16 steht, können nach bekannten Methoden hergestellt werden, beispielsweise analog der in US2009/203705, US2012/258951, WO2013/3298 oder J. Med. Chem. 31, (1988) 1590-1595 beschriebenen Verfahren.

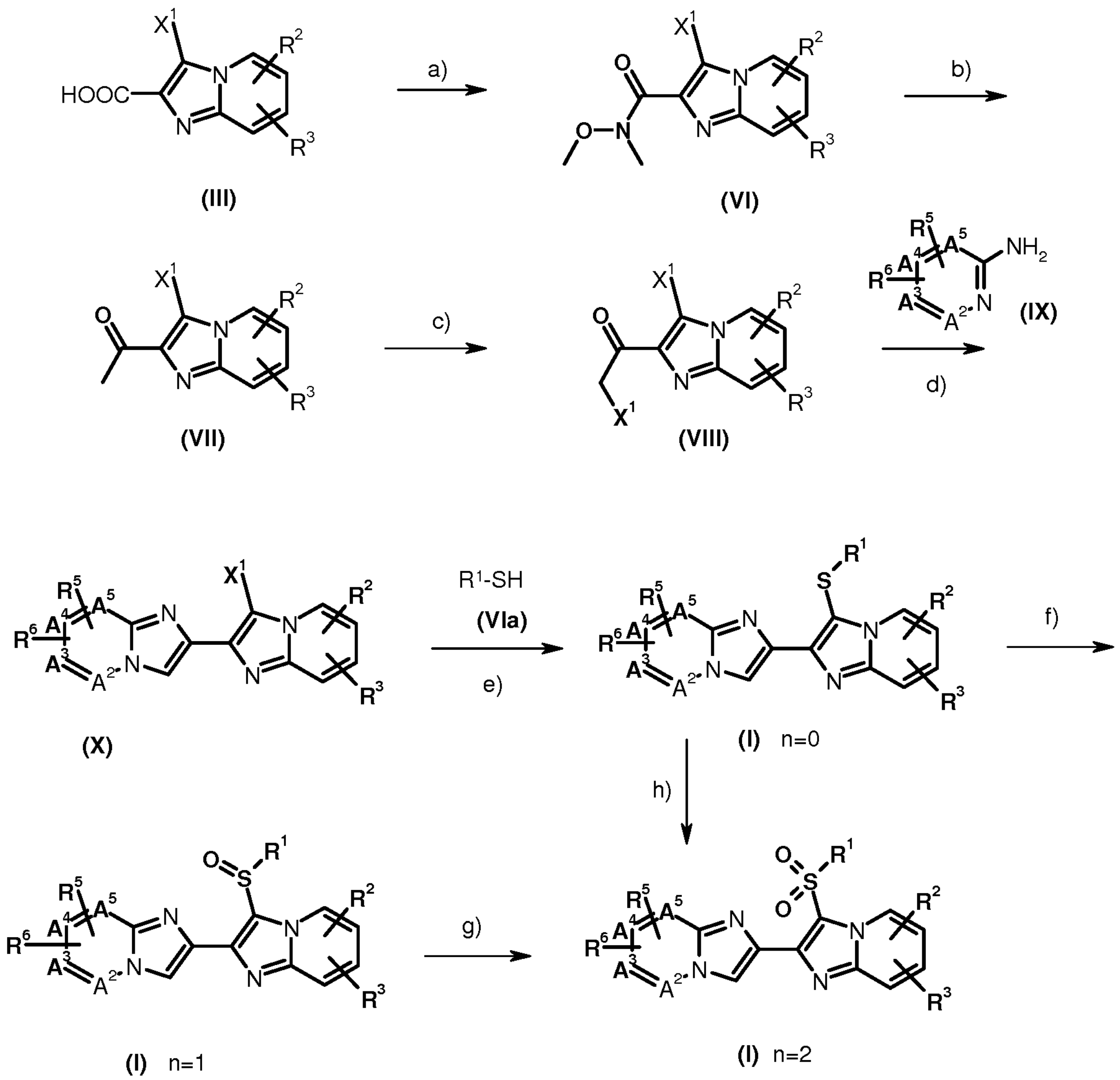


(III) (VI) (VII) (VIII) (IX) (X) (I) n=0 (I) n=1 (I) n=2

Die Reste $R^1$, $R^2$, $R^3$, $R^5$, $R^6$ und n haben die oben beschriebenen Bedeutungen. $A^2$, $A^3$, $A^4$ und $A^5$ stehen für CH oder N (wobei $A^2$, $A^3$, $A^4$ und $A^5$ nicht gleichzeitig für N stehen) und $X^1$ steht für Halogen.

**Schritt a)**

Carbonsäuren der Formel (III) werden in Analogie zu dem in WO2011/75643 oder EP2671582 beschriebenen Verfahren in Gegenwart von O,N-Dimethyl-hydroxylamin Hydrochlorid in Weinreb-Amide der Formel (VI) überführt.

Carbonsäuren der Formel (III) sind entweder kommerziell erhältlich oder können nach bekannten Methoden hergestellt werden, beispielsweise aus 2-Aminopyridin Derivaten analog der in WO2011/41713 beschriebenen Verfahren.

**Schritt b, c)**

Verbindungen der Formel (VI) lassen sich anschließend nach bekannten Methoden, beispielsweise analog dem in WO2011/75643 beschriebenen Verfahren, mit einem Grignard Reagenz wie beispielsweise Methylmagnesiumbromid in Ketone der Formel (VII) überführen. Durch anschließende Halogenierung analog der beispielsweise in US2012/302573 beschriebenen bekannten Methode sind Verbindungen der Formel (VIII) zugänglich.

**Schritt d)**

Die Verbindungen der Formel (X) lassen sich herstellen durch Cyclisierung der Verbindungen der Formel (VIII)

mit Aminen der Formel (IX). Die Cyclisierung erfolgt beispielsweise in Ethanol, Acetonitril oder N,N-Dimethylformamid nach bekannten Methoden analog der beispielsweise in WO2005/66177, WO2012/88411, WO2013/3298, US2009/203705, US2012/258951, WO2012/168733, WO2014/187762 oder J. Med. Chem. 31 (1988) 1590-1595 beschriebenen Verfahren.

Die Verbindungen der Formel (IX) sind kommerziell erhältlich.

**Schritt e)**

Die Verbindungen der Formel (I), wobei n für 0 steht, lassen sich herstellen durch Umsetzung der Verbindungen der Formel (X) mit den Verbindungen der Formel (VIa) in Gegenwart einer Base. Mercaptanderivate der Formel (VIa) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

**Schritt f, g)**

Die Verbindungen der Formel (I), wobei n für 1 steht, lassen sich herstellen durch Oxidation der Verbindungen der Formel (I), wobei n für 0 steht. Die Oxidation erfolgt nach bekannten Methoden mit einem geeigneten Oxidationsmittel wie bespielsweise Wasserstoffperoxid, meta-Chlorperbenzoesäure oder Natriumperiodat.

Die Verbindungen der Formel (I), wobei n für 2 steht, lassen sich herstellen durch Oxidation der Verbindungen der Formel (I), wobei n für 1 steht.

Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser. Beispiele für geeignete Oxidationsmittel sind Wasserstoffperoxid und meta-Chlorperbenzoesäure.

**Schritt h)**

Die Verbindungen der Formel (I), wobei n für 2 steht, lassen sich auch in einem einstufigen Prozess herstellen durch Oxidation der Verbindungen der Formel (I), wobei n für 0 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser. Beispiele für geeignete Oxidationsmittel sind Wasserstoffperoxid und meta-Chlorperbenzoesäure.

**<u>Verfahren C</u>**

Die Verbindungen der Formel (I), bei denen Q für Q17 steht, können nach bekannten Methoden hergestellt werden, beispielsweise analog der in WO2014/142292 beschriebenen Verfahren.

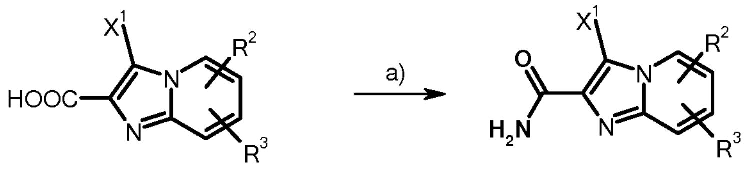


(III) (XI)

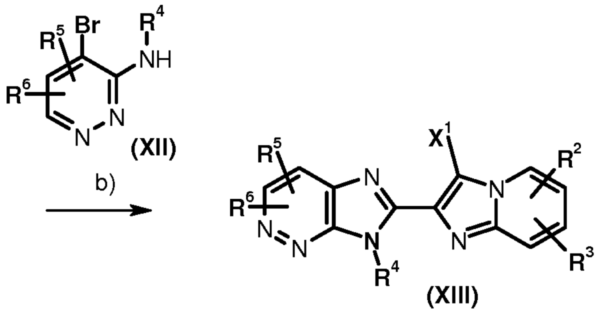


(XII) (XIII)

Die Reste $R^2$, $R^3$, $R^4$, $R^5$ und $R^6$ haben die oben beschriebenen Bedeutungen. $X^1$ steht für Halogen.

**Schritt a)**

Die Verbindungen der Formel (XI) können in Analogie zu dem in US5374646 oder Bioorganic and Medicinal Chemistry Letters 2003, 13, 1093 - 1096 beschriebenen Verfahren durch die Umsetzung von Verbindungen der Formel (III) mit einer Ammoniakquelle in Gegenwart eines Kondensationsmittels hergestellt werden.
Carbonsäuren der Formel (III) sind entweder kommerziell erhältlich oder können nach bekannten Methoden hergestellt werden, beispielsweise aus 2-Aminopyridin Derivaten analog der in WO2011/41713 beschriebenen Verfahren. Als Ammoniakquelle wird meist Ammoniumhydroxid verwendet.
Die Umsetzung der Verbindungen der Formel (III) mit der Ammoniakquelle wird vorzugsweise in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Dioxan oder Tetrahydrofuran.
Ein geeignetes Kondensationsmittel ist beispielsweise Carbonyldiimidazol.
Die Reaktion kann im Vakuum, bei Normaldruck oder unter Überdruck durchgeführt werden. Vorzugsweise erfolgt die Reaktion bei Normaldruck und Temperaturen von 20 bis 70 °C.

**Schritt b)**

Die Verbindungen der Formel (XIII) können in Analogie zu dem in WO2014/142292 beschriebenen Verfahren durch die Umsetzung von Verbindungen der Formel (XI) mit Verbindungen der Formel (XII) in Gegenwart eines Palladiumkatalysators im Basischen hergestellt werden.
Verbindungen der Formel (XII) können beispielsweise analog der in WO2014/142292 beschriebenen Verfahren hergestellt werden. Als Palladiumkatalysator kann beispielsweise [1,1'-Bis-(diphenylphosphino)ferrocen]dichlorpalladium(II) verwendet werden. Als Base finden häufig anorganische Basen wie Kaliumtertbutanolat Verwendung
Die Umsetzung erfolgt in einem Lösungsmittel. Häufig wird Toluol verwendet.
Die Reaktion kann im Vakuum, bei Normaldruck oder unter Überdruck durchgeführt werden. Vorzugsweise erfolgt die Reaktion bei Normaldruck und Temperaturen von 20 bis 110 °C.
Die weitere Umsetzung von Verbindungen der Formel (XIII) zu Verbindungen der Formel (I) erfolgt analog zu Verfahren A.

**Verfahren D**

Die Verbindungen der Formel (I), bei denen Q für Q14 steht, können nach bekannten Methoden hergestellt werden, beispielsweise analog der in WO2011/073149 beschriebenen Verfahren.

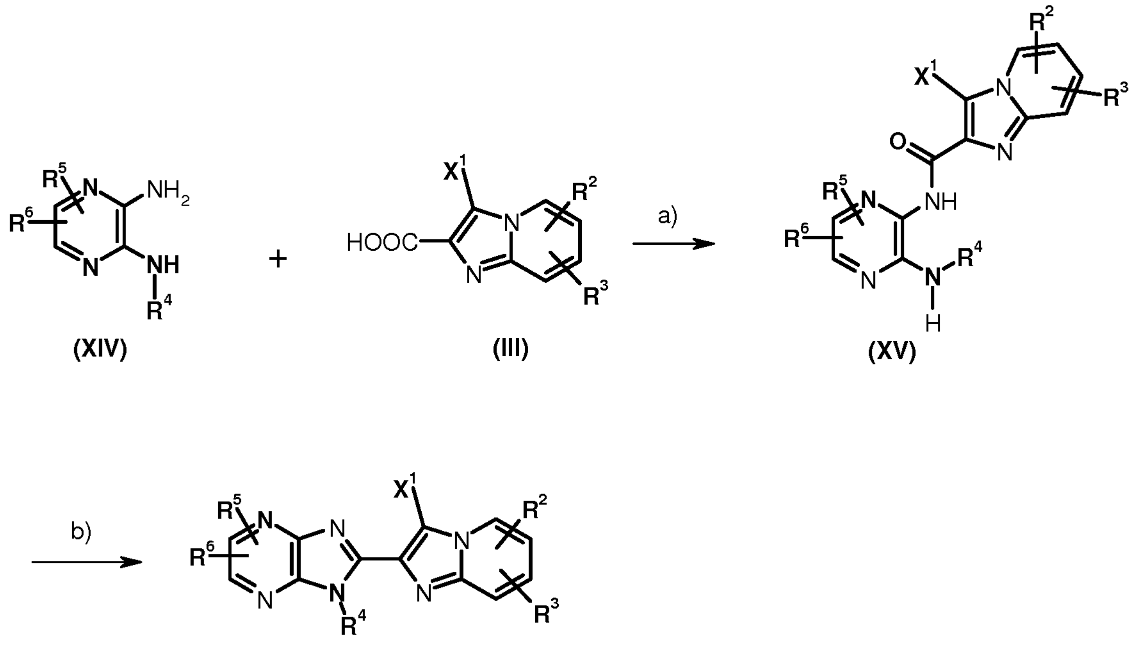


(XIV) (III) (XV)

(XVI)

Die Reste $R^2$, $R^3$, $R^4$, $R^5$ und $R^6$ haben die oben beschriebenen Bedeutungen. $X^1$ steht für Halogen.

**Schritt a)**

Die Verbindungen der Formel (XV) können in Analogie zu dem in WO2011/073149 oder US5576335 beschriebenen Verfahren durch die Umsetzung von Verbindungen der Formel (XIV) mit einer Carbonsäure der Formel (III) in Gegenwart eines Kondensationsmittels bzw. einer Base hergestellt werden.

Verbindungen der Formel (XIV) sind entweder kommerziell erhältlich oder können nach bekannten Methoden hergestellt werden, beispielsweise analog der in WO2008/51493 oder in Bioorganic and Medicinal Chemistry 2014, 22, 13, 3515 - 3526 beschriebenen Verfahren.

Carbonsäuren der Formel (III) sind entweder kommerziell erhältlich oder können nach bekannten Methoden hergestellt werden, beispielsweise aus 2-Aminopyridin Derivaten analog der in WO2011/41713 beschriebenen Verfahren.

Die Umsetzung der Verbindungen der Formel (XIV) mit Carbonsäuren der Formel (III) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI) oder 1,3-Dicyclohexylcarbodiimid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

**Schritt b)**

Die Verbindungen der Formel (XVI) lassen sich herstellen durch Kondensation der Verbindungen der Formel (XV) z.B. analog der in WO2009/131237, WO2010/125985, WO2011/043404, WO2011/040629, WO2012/086848,

WO2013/018928 oder WO2015/000715 beschriebenen Verfahren.

Die Umsetzung zu Verbindungen der Formel (XVI) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

Die weitere Umsetzung von Verbindungen der Formel (XVI) zu Verbindungen der Formel (I) erfolgt analog zu Verfahren A.

## Verfahren E

Die Verbindungen der Formel (I), bei denen Q für Q12, Q13, Q18 und Q19 steht, können nach bekannten Methoden hergestellt werden, beispielsweise analog der in WO2010/091310, WO 2012/66061 oder WO2013/099041 beschriebenen Verfahren.

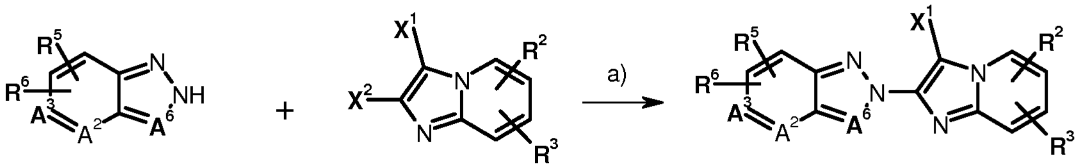


(XVII) (XVIII) (XIX)

Die Reste $R^2$, $R^3$, $R^5$ und $R^6$ haben die oben beschriebenen Bedeutungen. $A^2$, $A^3$ und $A^6$ stehen für CH oder N (wobei $A^2$ und $A^3$ nicht gleichzeitig für N stehen können). $X^1$ und $X^2$ stehen für Halogen.

### Schritt a)

Die Verbindungen der Formel (XIX) lassen sich herstellen durch Umsetzung von Verbindungen der Formel (XVII) mit Verbindungen der Formel (XVIII) unter basischen Bedingungen, z.B. analog der in WO2010/091310, WO 2012/66061 oder WO2013/099041 beschriebenen Verfahren.

Verbindungen der Formel (XVII) sind entweder kommerziell erhältlich oder können nach bekannten Methoden hergestellt werden, beispielsweise analog der in WO2005/100353, WO 2012/66061 oder in European Journal of Medicinal Chemistry 2010, 45, 2214 - 2222 beschriebenen Verfahren.

Verbindungen der Formel (XVIII) sind entweder kommerziell erhältlich oder können nach bekannten Methoden hergestellt werden, beispielsweise analog der in WO2013/43518, EP2168965 oder in Journal of Medicinal Chemistry 2003, 46, 1449 - 1455 beschriebenen Verfahren.

Als Basen werden meist anorganische Basen wie Natriumhydrid, Kaliumcarbonat oder Cäsiumcarbonat verwendet.

Die Umsetzung zu Verbindungen der Formel (XIX) erfolgt meist in einem Lösungsmittel, vorzugsweise in einem

Nitril, wie beispielsweise Acetonitril oder Propionitril oder in einem aprotischen, polaren Lösungsmittel wie beispielsweise N,N-Dimethylformamid oder N-Methylpyrrolidon.

Die Reaktion kann im Vakuum, bei Normaldruck oder unter Überdruck und bei Temperaturen von 0 °C bis 200 °C durchgeführt werden.

Alternativ kann die Umsetzung von Verbindungen der Formel (XVII) mit Verbindungen der Formel (XVIII) zu Verbindungen der Formel (XIX) auch durch Palladium-katalysierte *N*-Arylierung erfolgen, z.B. analog der in Angewandte Chemie Int. Ed. 2011, 50, 8944-8947 beschriebenen Verfahren.

Die weitere Umsetzung von Verbindungen der Formel (XIX) zu Verbindungen der Formel (I) erfolgt analog zu Verfahren A.

Verfahren F

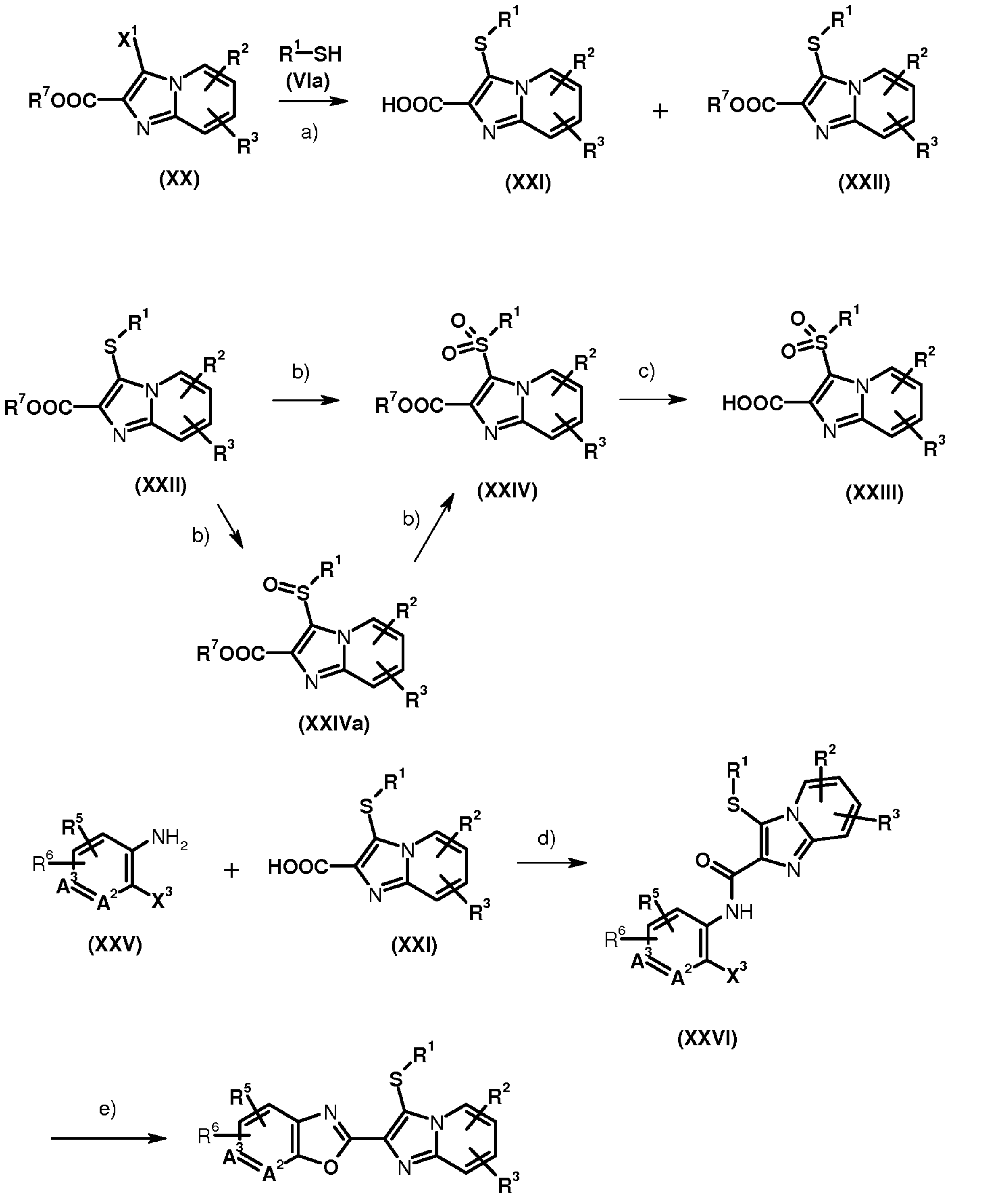


(XX) (XXI) (XXII)

(XXII) (XXIV) (XXIII)

(XXIVa)

(XXV) (XXI) (XXVI)

(I) n=0

Die Reste $R^1$, $R^2$, $R^3$, $R^5$, $R^6$ und n haben die oben beschriebenen Bedeutungen, $A^2$ und $A^3$ stehen für CH oder N, $X^1$ steht für Halogen, $X^3$ steht für Halogen und $R^7$ steht für $(C_1\text{-}C_4)$Alkyl.

**Schritt a)**

Die Verbindungen der Formeln (XXI) und (XXII) lassen sich herstellen durch Umsetzung der Verbindungen der Formel (XX) mit den Verbindungen der Formel (VIa) in Gegenwart einer Base.

Die Verbindungen der Formel (XX) sind entweder kommerziell erhältlich oder können nach bekannten Methoden hergestellt werden, beispielsweise aus 2-Aminopyridin Derivaten analog der in WO2011/41713 beschriebenen Verfahren.

Mercaptanderivate der Formel (VIa) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindung der Formeln (XXI) und (XXII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

**Schritt b)**

Die Verbindungen der Formel (XXIV) lassen sich herstellen durch Oxidation der Verbindungen der Formel (XXII). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure. Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C °C bis 120 °C durchgeführt werden.

Die Verbindungen der Formel (XXIVa) lassen sich analog durch Oxidation der Verbindungen der Formel (XXII) herstellen.

Die Verbindungen der Formel (XXIV) lassen sich analog durch Oxidation der Verbindungen der Formel (XXIVa) herstellen.

**Schritt c)**

Die Verbindungen der Formel (XXIII) lassen sich herstellen durch Verseifung der Verbindungen der Formel (XXIV) in Gegenwart einer Base. Die Verseifung wird generell in einem Lösungsmittel durchgeführt. Bevorzugt werden Alkohole wie Methanol oder Ethanol; Wasser; Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid; oder Gemische aus den genannten Lösungsmitteln.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat.

Die Reaktion kann im Vakuum, bei Normaldruck oder unter Überdruck und bei Temperaturen von -20°C °C bis 200 °C durchgeführt werden.

Die weitere Umsetzung von Verbindungen der Formel (XXIII) zu Verbindungen der Formel (I) erfolgt analog zu Verfahren A.

**Schritt d)**

Die Verbindungen der Formel (XXVI) können durch die Umsetzung von Verbindungen der Formel (XXV) mit Carbonsäuren der Formel (XXI) in Gegenwart eines Kondensationsmittels bzw. einer Base hergestellt werden.

Die Verbindungen der Formel (XXV) sind entweder kommerziell erhältlich oder können nach bekannten Methoden hergestellt werden, beispielsweise analog der in US2003/069257, US2012/0319050, WO2011/107998 oder WO2010/91310 beschriebenen Verfahren.

Die Umsetzung der Verbindungen der Formel (XXV) mit Carbonsäuren der Formel (XXI) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile,

wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI), 1,3-Dicyclohexylcarbodiimid, Thionylchlorid, oder Oxalylchlorid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

**Schritt e)**

Die Verbindungen der Formel (I), wobei n für 0 steht, lassen sich durch Kondensation der Verbindungen der Formel (XXVI) in Gegenwart einer Base herstellen.

Die Umsetzung zu Verbindungen der Formel (I), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 200 °C durchgeführt werden.

Gegenstand der Erfindung sind auch Verbindungen der Formel (XXII)

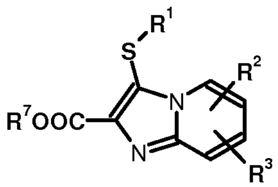

in welcher
$R^1$, $R^2$ und $R^3$ die oben angegebenen Bedeutungen haben und
$R^7$ für ($C_1$-$C_4$)Alkyl (bevorzugt für Methyl oder Ethyl, besonders bevorzugt für Ethyl) steht.

Gegenstand der Erfindung sind auch Verbindungen der Formel (XXIII)

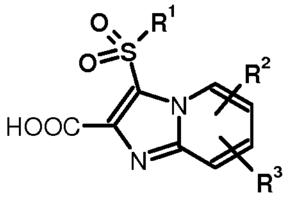

in welcher $R^1$, $R^2$ und $R^3$ die oben angegebenen Bedeutungen haben.

Gegenstand der Erfindung sind auch Verbindungen der Formel (XXIV)

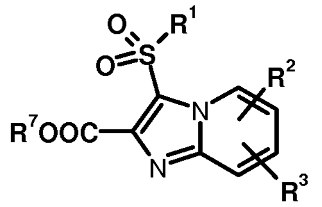

in welcher $R^1$, $R^2$, $R^3$ und $R^7$ die oben angegebenen Bedeutungen haben.

Gegenstand der Erfindung sind auch Verbindungen der Formel (XXIVa)

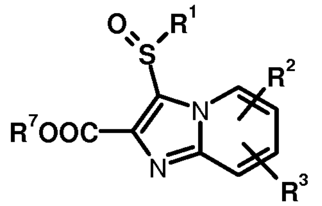

in welcher $R^1$, $R^2$, $R^3$ und $R^7$ die oben angegebenen Bedeutungen haben.

Gegenstand der Erfindung sind auch Verbindungen der Formel (XXVI)

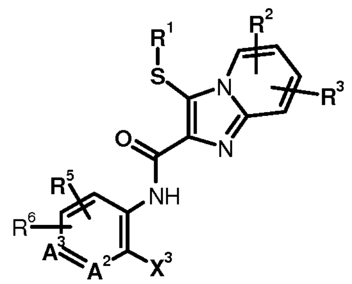

in welcher

$X^3$ für Halogen (bevorzugt für Chlor, Brom, Iod oder Fluor; besonders bevorzugt für Chlor oder Brom, ganz besonders bevorzugt für Chlor) steht,

$A^2$ und $A^3$ für CH oder N stehen,

$R^1$, $R^2$, $R^3$, $R^5$ und $R^6$ die oben angegebenen Bedeutungen haben.

Die Zwischenverbindungen der Formel (III) sind zum Teil bekannt aus: WO 2011/073316, WO 2013/018371, WO 2009/023179, WO 2011/041713.

Die Zwischenverbindungen der Formel (V) sind zum Teil bekannt aus: Archiv der Pharmazie, Bd. 335, Nr. 1, Seiten 27-32, Chemical and Pharmaceutical Bulletin, Bd. 39, Nr. 11, Seiten 2937-2943, EP 0120598 A1, EP 0052016 A1, The Journal of Organic Chemistry, Bd. 41, Nr. 22, Seiten 3549-3556.

Die Zwischenverbindungen der Formel (XXII) und der Formel (XXIV) sind zum Teil bekannt aus: WO 97/11075, Tetrahedron, Bd. 69, Nr. 38, Seiten 8255-8262, Chemometrics and Intelligent Laboratory Systems, Bd. 20, Nr, 1, Seiten 71-84, Asian Journal of Organic chemistry, Bd. 3, Nr. 5, Seiten 609-613, Journal of Organic Chemistry, Bd. 46, Nr, 5, Seiten 1026-1030, Chemical Abstracts Service XP002757605.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung wird unter dem Begriff "Hygiene" die Gesamtheit aller Maßnahmen, Verfahren und Verhaltensweisen verstanden, die das Ziel haben, Erkrankungen - insbesondere Infektionskrankheiten - zu vermeiden und der Gesunderhaltung des Menschen, Tieres und/oder der Umwelt zu dienen und/oder die Sauberkeit zu erhalten. Hierunter fallen erfindungsgemäß insbesondere Maßnahmen zur Reinigung, Desinfektion und Sterilisation beispielsweise von Textilien oder harten Oberflächen, vornehmlich aus Glas, Holz, Beton, Porzellan, Keramik, Kunststoff oder auch aus Metall(en), und deren Reinhaltung von Hygieneschädlingen bzw. deren Fäkalien. Erfindungsgemäß ausgeschlossen sind diesbezüglich wiederum Verfahren zur chirurgischen oder therapeutischen Behandlung des menschlichen oder tierischen Körpers und Diagnostizierverfahren, die am menschlichen oder tierischen Körper vorgenommen werden.

Der Begriff "Hygienesektor" umfasst demnach alle Bereiche, technischen Gebiete und gewerblichen Nutzungen, in denen derartige Maßnahmen, Verfahren und Verhaltensweisen zur Hygiene von Wichtigkeit sind, wie beispielsweise die Hygiene in Küchen, Bäckereien, Flughäfen, Bädern, Schwimmbädern, Einkaufshäusern, Hotels, Krankenhäusern, Stallungen, etc.

Unter dem Begriff "Hygieneschädling" werden folglich ein oder mehrere tierischer(e) Schädling(e) verstanden, deren Vorhandensein im Hygienesektor insbesondere aus gesundheitlichen Gründen problematisch ist. Es ist folglich das vornehmliche Ziel, Hygieneschädlinge bzw. den Kontakt mit ihnen im Hygienesektor zu vermeiden bzw. zu minimieren. Dies kann insbesondere durch die Anwendung eines Schädlingsbekämpfungsmittels erfolgen, wobei das Mittel sowohl prophylaktisch als auch erst bei Befall zur Bekämpfung des Schädlings eingesetzt werden kann. Es ist ebenfalls möglich, Mittel einzusetzen, die bewirken, dass ein Kontakt mit dem Schädling vermieden oder reduziert wird. Als Hygieneschädlinge kommen beispielsweise die nachstehend genannten Organismen in Betracht.

Der Begriff "Hygieneschutz" umfasst demnach alle Handlungen zur Aufrechterhaltung und/oder Verbesserung von derartigen Maßnahmen, Verfahren und Verhaltensweisen zur Hygiene.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;

aus der Klasse der Chilopoda z.B. Geophilus spp., Scutigera spp.;

aus der Ordnung oder der Klasse der Collembola z.B. Onychiurus armatus; Sminthurus viridis;

aus der Klasse der Diplopoda z.B. Blaniulus guttulatus;

aus der Klasse der Insecta, z.B. aus der Ordnung der Blattodea z.B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z.B. Periplaneta americana, Periplaneta australasiae, Pycnoscelus surinamensis, Supella

longipalpa;

aus der Ordnung der Coleoptera z.B. Acalymma vittatum, Acanthoscelides obtectus, Adoretus spp., Aethina tumida, Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Listronotus (= Hyperodes) spp., Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oulema spp., Oulema oryzae, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;

aus der Ordnung der Dermaptera z.B. Anisolabis maritime, Forficula auricularia, Labidura riparia;

aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex, Toxotrypana curvicauda;

aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lopholeucaspis japonica, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Neomaskellia spp., Nephotettix spp., z.B. Nephotettix cincticeps,, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Perkinsiella spp., Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pulvinaria spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;

aus der Unterordnung der Heteroptera z.B. Aelia spp., Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Megacopta cribraria, Miridae, Monalonion atratum, Nezara spp., z.B. Nezara viridula, Nysius spp., Oebalus spp., Pentomidae, Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;

aus der Ordnung der Hymenoptera z.B. Acromyrmex spp., Athalia spp., z.B. Athalia rosae, Atta spp., Camponotus spp., Dolichovespula spp., Diprion spp., z.B. Diprion similis, Hoplocampa spp., z.B. Hoplocampa cookei, Hoplocampa testudinea, Lasius spp., Linepithema humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z.B.

Vespa crabro, Wasmannia auropunctata, Xeris spp.;

aus der Ordnung der Isopoda z.B. Armadillidium vulgare, Oniscus asellus, Porcellio scaber;

aus der Ordnung der Isoptera z.B. Coptotermes spp., z.B. Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., z.B. Reticulitermes flavipes, Reticulitermes hesperus;

aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;

aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;

aus der Ordnung der Phthiraptera z.B. Damalinia spp., Haematopinus spp., Linognathus spp., Pediculus spp., Phylloxera vastatrix, Phthirus pubis, Trichodectes spp.;

aus der Ordnung der Psocoptera z.B. Lepinotus spp., Liposcelis spp.;

aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;

aus der Ordnung der Thysanoptera z.B. Anaphothrips obscurus, Baliothrips biformis, Chaetanaphothrips leeuweni, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Rhipiphorothrips cruentatus, Scirtothrips spp., Taeniothrips cardamomi, Thrips spp., z.B. Thrips palmi, Thrips tabaci;

aus der Ordnung der Zygentoma (= Thysanura), z. B. Ctenolepisma spp., Lepisma saccharina, Lepismodes inquilinus, Thermobia domestica;

aus der Klasse der Symphyla z.B. Scutigerella spp., z.B. Scutigerella immaculata;

Schädlinge aus dem Stamm der Mollusca, insbesondere aus der Klasse der Bivalvia, z.B. Dreissena spp.;

sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;

Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;

Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchulus spp., Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia, z.B. Eimeria spp., bekämpfen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

## Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropylguar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylenchlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate),

Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

**Mischungen**

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung

der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

**Insektizide / Akarizide / Nematizide**

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).

(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.

(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(IR)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Momfluorothrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.

(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.

(5) Nikotinerge Acetylcholin-Rezeptor (nAChR) allosterische Aktivatoren, wie beispielsweise Spinosine, z.B. Spinetoram und Spinosad.

(6) Chlorid-Kanal-Aktivatoren, wie beispielsweise Avermectine/Milbemycine, z.B. Abamectin, Emamectin-benzoat, Lepimectin und Milbemectin.

(7) Juvenilhormon-Imitatoren, wie beispielsweise Juvenilhormon-Analoge, z.B. Hydroprene, Kinoprene und Methoprene oder Fenoxycarb oder Pyriproxyfen.

(8) Wirkstoffe mit unbekannten oder nicht spezifischen Wirkmechanismen, wie beispielsweise Alkylhalide, z.B. Methylbromid und andere Alkylhalide; oderChloropicrin oder Sulfurylfluorid oder Borax oder Brechweinstein.

(9) Selektive Fraßhemmer, z.B. Pymetrozine oder Flonicamid.

(10) Milbenwachstumsinhibitoren, z.B. Clofentezine, Hexythiazox und Diflovidazin oder Etoxazole.

(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, CrylAc, CrylFa, Cry2Ab, mCry3A, Cry3Ab, Cry3Bb, Cry34/35Ab1.

(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.

(13) Entkoppler der oxidativen Phoshorylierung durch Unterbrechung des H-Protongradienten, wie beispielsweise Chlorfenapyr, DNOC und Sulfluramid.

(14) Nikotinerge Acetylcholin-Rezeptor-Antagonisten, wie beispielsweise Bensultap, Cartaphydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungshemmer (insbesondere bei Dipteren, d.h.Zweiflüglern), wie beispielsweise Cyromazine.

(18) Ecdyson-Rezeptor Agonisten, wie beispielsweise Chromafenozide, Halofenozide, Methoxyfenozide und Tebufenozide.

(19) Oktopaminerge Agonisten, wie beispielsweise Amitraz.

(20) Komplex-III-Elektronentransportinhibitoren, wie beispielsweise Hydramethylnon oder Acequinocyl oder Fluacrypyrim.

(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).

(22) Spannungsabhängige Natriumkanal-Blocker, z.B. Indoxacarb oder Metaflumizone.

(23) Inhibitoren der Acetyl-CoA-Carboxylase, wie beispielsweise Tetron- und Tetramsäurederivate, z.B. Spirodiclofen, Spiromesifen und Spirotetramat.

(24) Komplex-IV-Elektronentransportinhibitoren, wie beispielsweise Phosphine, z.B. Aluminiumphosphid, Calciumphosphid, Phosphin und Zinkphosphid oder Cyanid.

(25) Komplex-II-Elektronentransportinhibitoren, wie beispielsweise Cyenopyrafen und Cyflumetofen.

(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Broflanilide, Bromopropylate, Chinomethionat, Cryolite, Cyclaniliprole, Cycloxaprid, Cyhalodiamide Dicloromezotiaz, Dicofol, Diflovidazin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, Lotilaner, Meperfluthrin, Paichongding, Pyflubumide, Pyridalyl, Pyrifluquinazon, Pyriminostrobin, Sarolaner, Tetramethylfluthrin, Tetraniliprole, Tetrachlorantraniliprole, Tioxazafen, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851),

4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden]-2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormethyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, Butyl-[2-(2,4-dichlorphenyl)-3-oxo-4-oxaspiro[4.5]dec-1-en-1-yl]-carbonat (bekannt aus CN 102060818), 3E)-3-[1-[(6-Chlor-3-pyridyl)methyl]-2-pyridyliden]-1,1,1-trifluor-propan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamid bekannt aus WO2010/051926).

**Fungizide**

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.

1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazol-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion,

(1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,SR)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.

2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von syn-epimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{ [4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.

3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylacetamid, (3.24) (2E)-2-(Methoxyimino)-N-methyl-2-(2-{[({(1E)-1-[3-(trifluormethyl)phenyl]ethyliden}-amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.

4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.

5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.

6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.

7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.

8) Inhibitoren der ATP-Produktion, zum Beispiel (8.01) Fentinacetat, (8.02) Fentinchlorid, (8.03) Fentinhydroxid, (8.04) Silthiofam.

9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.

10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.

11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.

12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.

13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.

14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.

15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothalisopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2- [(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-l-yloxy)phenyl]propanamid, (15.078) N-[(5-Brom-3-chlorpyridin-2-yl)methyl]-2,4-dichlornicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}carbamidsäurepentylester, (15.088) Phenazin-1-carbonsäure, (15.089) Chinolin-8-ol, (15.090) Chinolin-8-olsulfat

(2:1), (15.091) {6-[({[(1-Methyl-1H-tetrazol-5-yl)(phenyl)methylen]amino}oxy)methyl]pyridin-2-yl}carbamidsäure-tert.-butylester, (15.092) (5-Brom-2-methoxy-4-methylpyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

**Biologische Schädlingsbekämpfungsmittel als Mischungspartner**

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:

*Bacillus amyloliquefaciens*, Stamm FZB42 (DSM 231179), oder *Bacillus cereus*, insbesondere *B. cereus* Stamm CNCM I-1562 oder *Bacillus firmus,* Stamm I-1582 (Accession number CNCM I-1582) oder *Bacillus pumilus*, insbesondere Stamm GB34 (Accession No. ATCC 700814) und Stamm QST2808 (Accession No. NRRL B-30087), oder *Bacillus subtilis*, insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis*, insbesondere *B. thuringiensis* subspecies *israelensis* (serotype H-14), Stamm AM65-52 (Accession No. ATCC 1276), oder *B. thuringiensis subsp. aizawai*, insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans*, *Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:

*Beauveria bassiana*, insbesondere Stamm ATCC 74040, *Coniothyrium minitans*, insbesondere Stamm CON/M/91-8 (Accession No. DSM-9660), *Lecanicillium spp*., insbesondere Stamm HRO LEC 12, *Lecanicillium lecanii*, (ehemals bekannt als *Verticillium lecanii*), insbesondere Stamm KV01, *Metarhizium anisopliae*, insbesondere Stamm F52 (DSM3884/ ATCC 90448), *Metschnikowia fructicola*, insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus*, insbesondere *P. lilacinus* Stamm 251 (AGAL 89/030550), *Talaromyces flavus*, insbesondere Stamm V117b, *Trichoderma atroviride*, insbesondere Stamm SC1 (Accession Number CBS 122089), *Trichoderma harzianum*, insbesondere *T. harzianum rifai T39.* (Accession Number CNCM I-952).

Beispiele für Viren, die als biologische Schädlingsbekämpfungsmittel eingesetzt werden bzw. verwendet werden können, sind:

*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:

*Agrobacterium spp., Azorhizobium caulinodans, Azospirillum spp*., *Azotobacter spp., Bradyrhizobium spp*., *Burkholderia spp.,* insbesondere *Burkholderia cepacia* (ehemals bekannt als *Pseudomonas cepacia*), *Gigaspora spp*., oder *Gigaspora monosporum*, *Glomus spp*., *Laccaria spp., Lactobacillus buchneri*, *Paraglomus spp*., *Pisolithus tinctorus*, *Pseudomonas spp*., *Rhizobium spp*., insbesondere *Rhizobium trifolii, Rhizopogon spp*., *Scleroderma spp*., *Suillus spp*., *Streptomyces spp.*.

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:

Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

**Safener als Mischpartner**

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

**Pflanzen und Pflanzenteile**

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

**Transgene Pflanze, Saatgutbehandlung und Integrationsereignisse**

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonyl-harnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

**Pflanzenschutz - Behandlungsarten**

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

**Saatgutbehandlung**

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze

bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das

Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den

Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

**Tiergesundheit**

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

Zu den Arthropoden zählen:

aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;

aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

Weiterhin zählen zu den Arthropoden:

Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu parasitären Protozoen zählen:

Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;

Sarcomastigophora (Rhizopoda), wie Entamoebidae, zum Beispiel Entamoeba histolytica, Hartmanellidae, zum Beispiel Acanthamoeba sp., Harmanella sp.;

Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:

Monogenea: z.B.: Gyrodactylus spp., Dactylogyrus spp., Polystoma spp.;

Cestodes: aus der Ordnung Pseudophyllidea zum Beispiel: Diphyllobothrium spp., Spirometra spp., Schistocephalus spp., Ligula spp., Bothridium spp., Diplogonoporus spp.;

aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;

Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;

Rundwürmer: Trichinellida zum Beispiel: Trichuris spp., Capillaria spp., Paracapillaria spp., Eucoleus spp., Trichomosoides spp., Trichinella spp.;

aus der Ordnung Tylenchida zum Beispiel: Micronema spp., Strongyloides spp.;

aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;

aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;

Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;

aus der Ordnung Echinorhynchida zum Beispiel Acanthocephalus spp., Echinorhynchus spp., Leptorhynchoides spp.;

Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

**Anthelminthische Mischungspartner**

Beispielhaft seien folgende anthelmintische Mischungspartner genannt:
Anthelminthische Wirkstoffe, einschließlich trematicide und cestocide Wirkstoffe:

aus der Klasse der **macrocyclischen Lactone,** z. B.: Abamectin, Doramectin, Emamectin, Eprinomectin, Ivermectin, Milbemycin, Moxidectin, Nemadectin, Selamectin;

aus der Klasse der **Benzimidazole und Probenzimidazole,** z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;

aus der Klasse der **Cyclooctadepsipeptide,** z. B.: Emodepsid, PF1022;

aus der Klasse der **Aminoacetonitril-Derivate,** z. B.: Monepantel;

aus der Klasse der **Tetrahydropyrimidine,** z. B.: Morantel, Pyrantel, Oxantel;

aus der Klasse der **Imidazothiazole,** z. B.: Butamisol, Levamisol, Tetramisol;

aus der Klasse der **Salicylanilide,** z. B.: Bromoxanid, Brotianid, Clioxanid, Closantel, Niclosamid, Oxyclozanid, Rafoxanid, Tribromsalan;

aus der Klasse der **Paraherquamide,** z. B.: Derquantel, Paraherquamid;

aus der Klasse der **Aminophenylamidine,** z. B.: Amidantel, deacyliertes Amidantel (dAMD), Tribendimidin;

aus der Klasse der **Organophosphate,** z. B.: Coumaphos, Crufomat, Dichlorvos, Haloxon, Naphthalofos, Trichlorfon;

aus der Klasse der **substituierten Phenole,** z. B.: Bithionol, Disophenol, Hexachlorophen, Niclofolan, Meniclopholan, Nitroxynil;

aus der Klasse der **Piperazinone,** z. B.: Praziquantel, Epsiprantel;

aus anderen **diversen Klassen,** z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

**Vektorkontrolle**

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.
Beispiele für Vektoren und die von ihnen übertragenen Krankheiten bzw. Krankheitserreger sind:

1) Mücken

- Anopheles: Malaria, Filariose;
- Culex: Japanische Encephalitis, Filariasis, weitere virale Erkrankungen, Übertragung von Würmern;
- Aedes: Gelbfieber, Dengue-Fieber, Filariasis, weitere virale Erkrankungen;
- Simulien: Übertragung von Würmern insbesondere Onchocerca volvulus;

2) Läuse: Hautinfektionen, Fleckfieber (epidemic typhus);

3) Flöhe: Pest, endemisches Fleckfieber;

4) Fliegen: Schlafkrankheit (Trypanosomiasis); Cholera, weitere bakterielle Erkrankungen;

5) Milben: Acariose, Fleckfieber, Rickettsipocken, Tularämie, Saint-Louis-Enzephalitis, virale Hirnhautentzündung (FSME), Krim-Kongo-Fieber, Borreliose;

6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.
Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.
Eine Vektorkontrolle ist auch möglich, wenn die Verbindungen der Formel (I) Resistenz-brechend sind.
Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

**Schutz von technischen Materialen**

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

**Bekämpfung von tierischen Schädlingen auf dem Hygienesektor**

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

**Herstellungsbeispiele:**

**2-(3-Ethylsulfonylimidazo[1,2-a]pyridin-2-yl)-3-methyl-6-(trifluormethyl)-imidazo[4,5c]pyridin (I-1)**

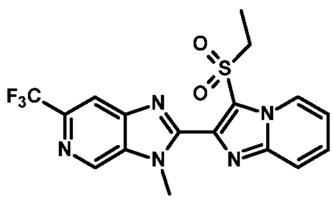

52 mg (0,13 mmol) 2-(3-Ethylsulfanylimidazo[1,2-a]pyridin-2-yl)-3-methyl-6-(trifluormethyl)-imidazo[4,5-c]pyridin wurden in 15 ml Dichlormethan gelöst, bei Raumtemperatur 31,8 mg (0,68 mmol) Ameisensäure und 133,9 mg (1,37 mmol) Wasserstoffperoxid zugegeben und anschließend 8 h bei Raumtemperatur gerührt. Der Ansatz wurde mit Wasser verdünnt und mit Natriumbisulfit-Lösung versetzt, 10 min gerührt, und anschließend mit 10%-iger Natriumhydrogencarbonat Lösung gerührt. Die organische Phase wurde abgetrennt, die wässrige Phase zweimal mit Dichlormethan extrahiert und die vereinigten organischen Phasen anschließend unter Vakuum vom Lösungsmittel befreit. Der Rückstand wurde durch säulenchromatographische Aufreinigung über präparative HPLC mit einem Wasser / Acetonitril Gradienten als Laufmittel gereinigt.
(logP (neutral): 2,07; $MH^+$: 410; $^1H$-NMR(400 MHz, $D_6$-DMSO) $\delta$ ppm: 1,28 (t, 3H), 3,87 (q, 2H), 4,08 (s, 3H), 7,41 (t, 1H), 7.75-7,79 (m, 1H), 7,99 (d, 1H), 8,29 (s, 1H), 9,07 (d, 1H), 9,28 (s, 1H).

**2-(3-Ethylsulfanylimidazo[1,2-a]pyridin-2-yl)-3-methyl-6-(trifluormethyl)-imidazo[4,5c]pyridin (I-2)**

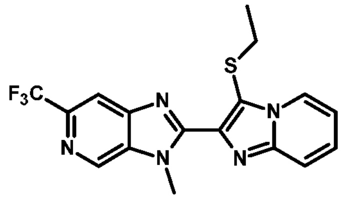

101 mg (0,28 mmol) 2-(3-Chlorimidazo[1,2-a]pyridin-2-yl)-3-methyl-6-(trifluormethyl)imidazo[4,5-c]pyridin und 73 mg (0,86 mmol) Natrium-Ethanthiolat wurden 8 h bei Raumtemperatur in DMF gerührt. Der Ansatz wurde mit Wasser versetzt und zweimal mit Essigester extrahiert. Die vereinigten organischen Phasen wurden mit einer Natriumchlorid Lösung gewaschen, abgetrennt, über Natriumsulfat getrocknet und im Vakuum vom Lösungsmittel befreit.
(logP (neutral): 2,75; $MH^+$: 378; $^1H$-NMR(400 MHz, $D_6$-DMSO) $\delta$ ppm: 1,08 (t, 3H), 3,04 (q, 2H), 4,29 (s, 3H), 7,24 (t, 1H), 7.54-7,58 (m, 1H), 7,83 (d, 1H), 8,26 (s, 1H), 8,79 (d, 1H), 9,22 (s, 1H).

**2-(3-Chlorimidazo[1,2-alpyridin-2-yl)-3-methyl-6-(trifluormethyl)imidazo[4,5-c]pyridin (V-1)**

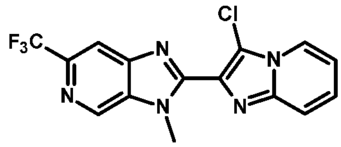

250 mg (1,30 mmol) $N^3$-Methyl-6-(trifluormethyl)pyridin-3,4-diamin, 305 mg (1,30 mmol) 3-Chlorimidazo[1,2-a]pyridin-2-carbonsäure-hydrochlorid und 251 mg (1,30 mmol) 1-(3-Dimethylaminopropyl)-3-ethylcarbodiimid hydrochlorid (EDCI) wurden in 5 ml Pyridin 9 h bei 120 °C gerührt. Das Reaktionsgemisch wurde im Vakuum vom Lösungsmittel befreit, anschließend Wasser zugegeben und dreimal mit Essigester extrahiert. Die vereinigten organischen Phasen wurden über Natriumsulfat getrocknet, erneut eingeengt und durch säulenchromatographische Aufreinigung über präparative HPLC mit einem Wasser / Acetonitril Gradienten als Laufmittel gereinigt.
(logP (neutral): 2,38; $MH^+$: 352; $^1H$-NMR(400 MHz, $D_6$-DMSO) $\delta$ ppm: 4,42 (s, 3H), 7,26 (t, 1H), 7.52-7,57 (m, 1H), 7,84 (d, 1H), 8,27 (s, 1H), 8,54 (d, 1H), 9,22 (s, 1H).

**6-[3-(Ethylsulfonyl)imidazo[1,2-a]pyridin-2-yl]-2,2-difluor-5-methyl-5H-[1,3]dioxolo[4,5-f]benzimidazol (I-41)**

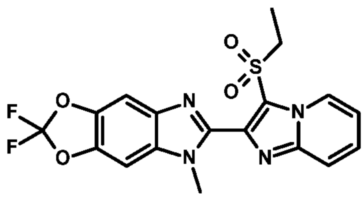

48 mg (0,11 mmol) 6-[3-(Ethylsulfonyl)imidazo[1,2-a]pyridin-2-yl]-2,2-difluor-5H-[1,3]dioxolo[4,5-f]benzimidazol wurden in 5 ml Aceton gelöst, bei Raumtemperatur 32,7 mg (0,23 mmol) Kaliumcarbonat und 23,5 mg (0,16 mmol) Iodmethan zugegeben und anschließend 4 h auf 60 °C erhitzt. Das Lösungsmittel wurde im Vakuum entfernt, der Rückstand in Essigester gelöst und die organische Phase mit Wasser gewaschen. Die wässrige Phase wurde zweimal mit Essigester extrahiert und die vereinten organischen Phasen über Natriumsulfat getrocknet, anschließend das Lösungsmittel im Vakuum entfernt. Das Rohprodukt wurde mittels MPLC mit einem Cyclohexan / Essigester Gradienten als Laufmittel gereinigt.
(logP (neutral): 2,62; $MH^+$: 421; $^1H$-NMR(600 MHz, $CD_3CN$) $\delta$ ppm: 1,32 (t, 3H), 3,82 (q, 2H), 3,92 (s, 3H), 7,20-7,22

(m, 1H), 7,42 (s, 1H), 7,50 (s, 1H), 7,61-7,64 (m, 1H), 7,78-7,80 (m, 1H), 9,10-9,12 (m, 1H).

**6-[3-(Ethylsulfonyl)imidazo[1,2-a]pyridin-2-yl]-2,2-difluor-5H-[1,3]dioxolo[4,5-f]benzimidazol (I-40)**

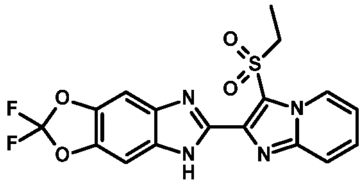

Zu einer Lösung von 57 mg (0,22 mmol) 3-(Ethylsulfonyl)imidazo[1,2-a]pyridin-2-carbonsäure in 3 ml Tetrahydrofuran und 3 ml Dimethylformamid wurde 128 mg (0,33 mmol) 1-[Bis(dimethylamino)methylen]-1*H*-1,2,3-triazolo[4,5-*b*]pyridinium-3-oxid Hexafluorphosphat (HATU) und 115 mg (0,89 mmol) *N*-Ethyldiisopropylamin gegeben und das Gemisch für 15 min bei Raumtemperatur gerührt. Anschließend wurde eine Lösung von 42,1 mg (0,22 mmol) 2,2-Difluor-1,3-benzodioxol-5,6-diamin in 3 ml Tetrahydrofuran zu getropft und die Reaktionsmischung über Nacht bei Raumtemperatur gerührt. Das Gemisch wurde mit Wasser versetzt und dreimal mit Essigester extrahiert. Die vereinten organischen Phasen wurden über Natriumsulfat getrocknet und anschließend das Lösungsmittel entfernt. Der Rückstand wurde in 20 ml Toluol gelöst und 91,2 mg (0,48 mmol) *p*-Toluolsulfonsäure, sowie Molsieb zugegeben. Die Reaktionsmischung wurde für 2 h auf 120 °C erhitzt. Das Gemsich wurde mit Wasser verdünnt und dreimal mit Essigester extrahiert. Die vereinten organischen Phasen wurden zweimal mit Natriumhydrogencarbonatlösung, einmal mit Natriumchloridlösung gewaschen, über Natriumsulfat getrocknet und anschließend das Lösungsmittel entfernt. Das Rohprodukt wurde mittels HPLC mit einem Wasser / Acetonitril Gradienten als Laufmittel gereinigt.
(logP (neutral): 2,59; $MH^+$: 407; $^1$H-NMR(400 MHz, $D_6$-DMSO) $\delta$ ppm: 1,28 (t, 3H), 4,17 (q, 2H), 7,31-7,34 (m, 1H), 7,53 (s, 1H), 7,68-7,72 (m, 1H), 7,77 (s, 1H), 7,91 (d, 1H), 9,21 (d, 1H).

**2-[3-(Ethylsulfonyl)imidazo[1,2-a]pyridin-2-yl]-6-(trifluormethyl)[1,3]oxazolo[5,4-b]pyridin (I-44) gilt als Referenzverbindung für die Herstellung**

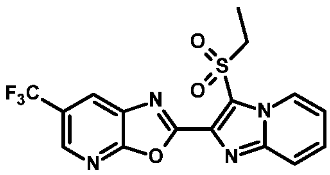

21 mg (0,05 mmol) 2-[3-(Ethylsulfanyl)imidazo[1,2-a]pyridin-2-yl]-6-(trifluormethyl)[1,3]oxazolo[5,4-b]pyridin wurden in 10 ml Dichlormethan gelöst, bei 0 °C 29,8 mg (0,17 mmol) 3-Chlorbenzolcarboperoxosäure (MCPBA) zugegeben und anschließend 4 h bei Raumtemperatur gerührt. Der Ansatz wurde mit Wasser verdünnt und mit Natriumbisulfit-Lösung versetzt, 10 min gerührt, und anschließend mit 10%-iger Natriumhydrogencarbonat Lösung gerührt. Die organische Phase wurde abgetrennt, die wässrige Phase zweimal mit Dichlormethan extrahiert und die vereinigten organischen Phasen anschließend unter Vakuum vom Lösungsmittel befreit. Der Rückstand wurde durch säulenchromatographische Aufreinigung über präparative HPLC mit einem Wasser / Acetonitril Gradienten als Laufmittel gereinigt.
(logP (neutral): 2,52; $MH^+$: 397; $^1$H-NMR(400 MHz, $D_6$-DMSO) $\delta$ ppm: 1,33 (t, 3H), 3,92 (q, 2H), 7,40-7,44 (m, 1H), 7,76-7,80 (m, 1H), 8,02 (d, 1H), 8,97-8,98 (m, 2H), 9,13 (d, 1H).

**2-[3-(Ethylsulfanyl)imidazo[1,2-a]pyridin-2-yl]-6-(trifluormethyl)[1,3]oxazolo[5,4-b]pyridin (I-60) gilt als Referenzverbindung für die Herstellung**

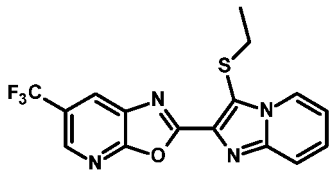

Zu einer Lösung von 39,8 mg (0,09 mmol) N-[2-Chlor-5-(trifluormethyl)pyridin-3-yl]-3-(ethylsulfanyl)imidazo[1,2-a]pyridin-2-carboxamid in 1 ml N,N-Dimethylformamid wurde 10,5 mg (0,09 mmol) Natriumcarbonat gegeben und das Gemisch für 4 h auf 145 °C erhitzt. Die Reaktionsmischung wurde auf Raumtemperatur abgekühlt, auf Eiswasser gegossen und zweimal mit Essigester extrahiert. Die vereinten organischen Phasen wurden über Natriumsulfat getrocknet und anschließend das Lösungsmittel entfernt. Das Rohprodukt wurde mittels HPLC mit einem Wasser / Acetonitril Gradienten als Laufmittel gereinigt.
(logP (neutral): 3,15; $MH^+$: 365; $^1$H-NMR(400 MHz, $D_6$-DMSO) $\delta$ ppm: 1,11 (t, 3H), 3,03 (q, 2H), 7,23-7,28 (m, 1H), 7,55-7,59 (m, 1H), 7,82 (d, 1H), 8,77 (d, 1H), 8,87 (s, 2H).

**N-[2-Chlor-5-(trifluormethyl)pyridin-3-yl]-3-(ethylsulfanyl)imidazo[1,2-a]pyridin-2-carboxamid (XXVI-1)**

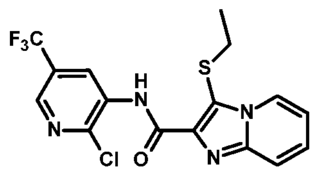

Zu einer Lösung von 298 mg (1,33 mmol) 3-(Ethylsulfanyl)imidazo[1,2-a]pyridin-2-carbonsäure in 10 ml Acetonitril wurde 1,1 mg (9,37 mmol) Thionylchlorid getropft und das Gemisch für 3 h refluxiert. Das Lösungsmittel wurde im Vakuum entfernt und zweimal mit Toluol co-evaporiert. Der Rückstand wurde in 3 ml N,N-Dimethylformamid gelöst. Getrennt davon wurde 263 mg (1,33 mmol) 2-Chlor-5-(trifluormethyl)pyridin-3-amin zu einer 0 °C kalten Suspension von 107 mg (2,67 mmol) Natriumhydrid in 7 ml N,N-Dimethylformamid gegeben und das Gemisch für 30 min nachgerührt. Anschließend wurde die vorher hergestellte Säurechloridlösung bei 0 °C langsam zugetropft. Die Reaktionsmischung wurde 2 h bei Raumtemperatur nachgerührt. Das Gemisch wurde auf Eiswasser gegossen und zweimal mit Essigester extrahiert. Die vereinten organischen Phasen wurden über Natriumsulfat getrocknet und anschließend das Lösungsmittel entfernt. Das Rohprodukt wurde mittels HPLC mit einem Wasser / Acetonitril Gradienten als Laufmittel gereinigt.
(logP (neutral): 4,39; $MH^+$: 401; $^1$H-NMR(400 MHz, $D_6$-DMSO) $\delta$ ppm: 1,09 (t, 3H), 2,98 (q, 2H), 7,22-7,25 (m, 1H), 7,55-7,60 (m, 1H), 7,82 (d, 1H), 8,66 (d, 1H), 8,73 (d, 1H), 9,07 (d, 1H).

In Analogie zu den Beispielen und gemäß den oben beschriebenen Herstellverfahren lassen sich folgende Verbindungen der Formel (I) erhalten:

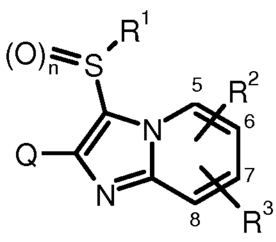

**(I)**

wobei $R^3$ für Wasserstoff steht und wobei $R^1$, $R^2$, Q und n, die in der folgenden Tabelle angegebenen Bedeutungen haben und wobei die Bindung von Q zum Restmolekül mit einer Wellenlinie gekennzeichnet ist:

| Bsp. | $R^1$ | n | $R^2$ | Q |
|---|---|---|---|---|
| I-1 | $C_2H_5$ | 2 | H | 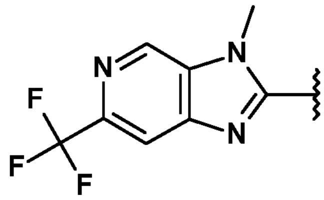 |
| I-2 | $C_2H_5$ | 0 | H | 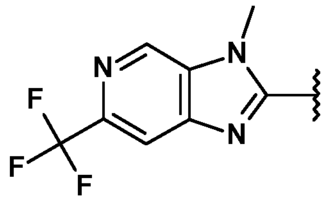 |
| I-7 | $C_2H_5$ | 1 | 6-Cl | 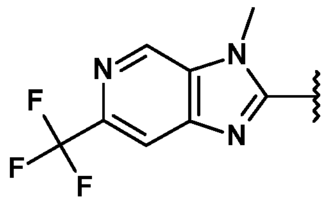 |
| I-8 | $C_2H_5$ | 1 | 6-$CF_3$ | 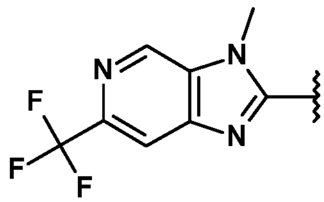 |
| I-9 | $C_2H_5$ | 0 | H | 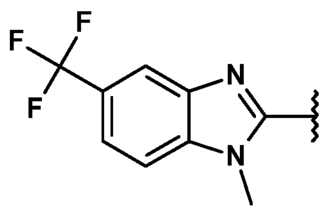 |
| I-10 | $C_2H_5$ | 2 | H | 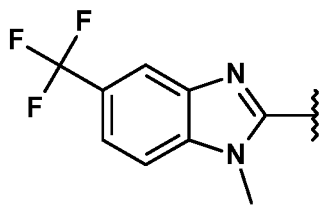 |
| I-11 | $C_2H_5$ | 1 | H | 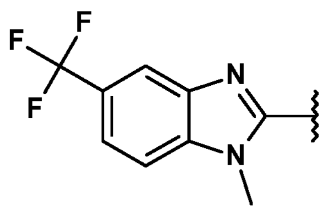 |

(fortgesetzt)

| Bsp. | $R^1$ | n | $R^2$ | Q |
|---|---|---|---|---|
| I-13 | $C_2H_5$ | 0 | 8-$CF_3$ | 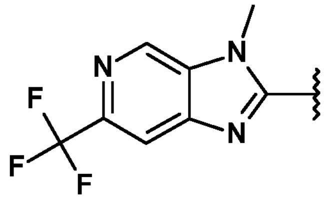 |
| I-14 | $C_2H_5$ | 0 | 8-Cl | 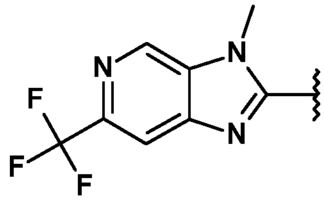 |
| I-15 | $C_2H_5$ | 0 | 8-$SC_2H_5$ | 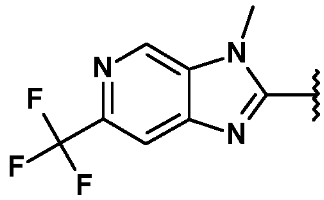 |
| I-18 | $C_2H_5$ | 1 | 7-$CF_3$ | 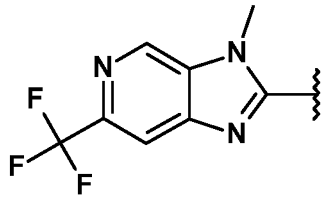 |
| I-19 | $C_2H_5$ | 2 | 8-Cl | 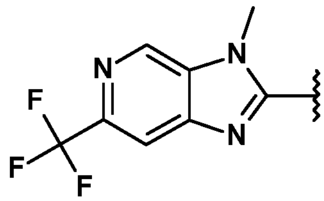 |
| I-20 | $C_2H_5$ | 0 | 5-$CF_3$ | 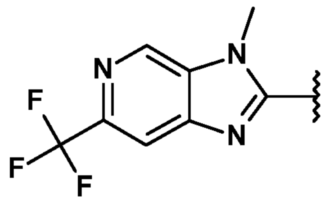 |
| I-21 | $C_2H_5$ | 2 | 5-$CF_3$ | 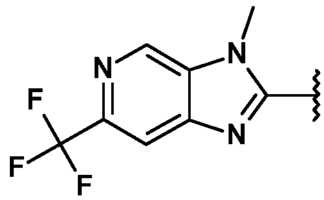 |

(fortgesetzt)

| Bsp. | $R^1$ | n | $R^2$ | Q |
|---|---|---|---|---|
| I-22 | $C_2H_5$ | 1 | 5-$CF_3$ | 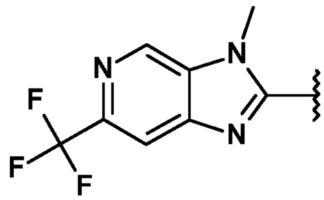 |
| I-24 | $C_2H_5$ | 2 | 8-$CF_3$ | 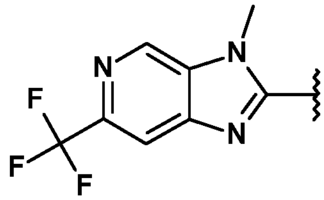 |
| I-25 | $C_2H_5$ | 1 | 8-$CF_3$ | 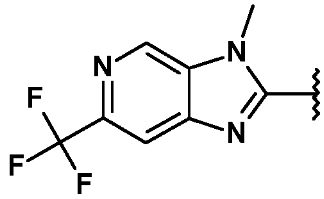 |
| I-26 | $C_2H_5$ | 0 | 5-$CH_3$ | 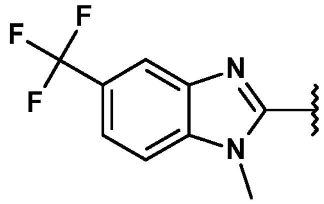 |
| I-27 | $C_2H_5$ | 0 | 5-$CF_3$ | 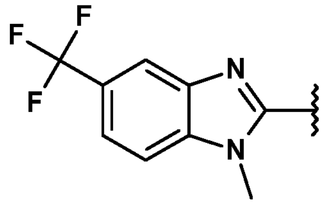 |
| I-28 | $C_2H_5$ | 0 | 6-$CF_3$ | 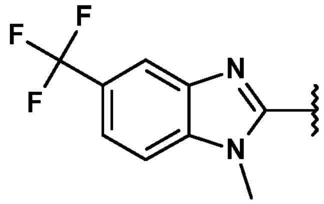 |
| I-29 | $C_2H_5$ | 0 | 6-Cl | 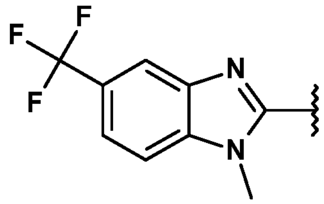 |

(fortgesetzt)

| Bsp. | $R^1$ | n | $R^2$ | Q |
|---|---|---|---|---|
| I-30 | $C_2H_5$ | 0 | 7-$CF_3$ | 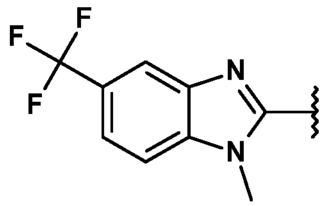 |
| I-31 | $C_2H_5$ | 0 | 5-$SC_2H_5$ | 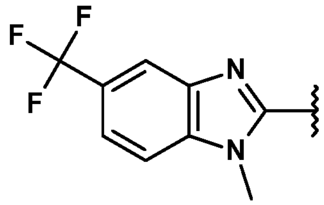 |
| I-32 | $C_2H_5$ | 0 | 7-$SC_2H_5$ | 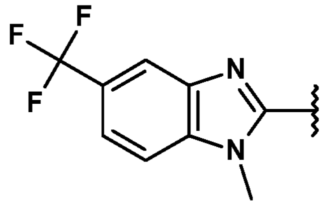 |
| I-33 | $C_2H_5$ | 2 | 7-$CH_3$ | 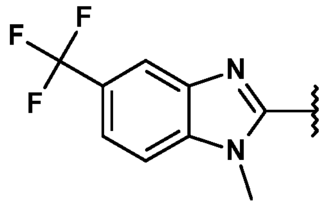 |
| I-34 | $C_2H_5$ | 2 | 7-$CF_3$ | 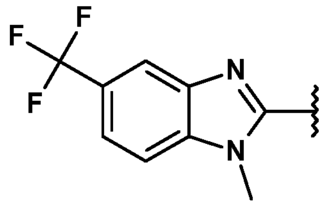 |
| I-35 | $C_2H_5$ | 2 | 6-$CH_3$ | 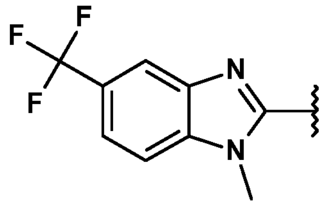 |
| I-36 | $C_2H_5$ | 2 | 6-$CF_3$ | 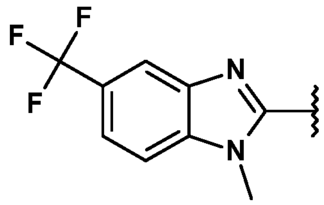 |

(fortgesetzt)

| Bsp. | $R^1$ | n | $R^2$ | Q |
|---|---|---|---|---|
| I-37 | $C_2H_5$ | 0 | 7-$CH_3$ | 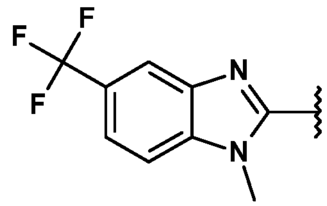 |
| I-38 | $C_2H_5$ | 0 | 6-$CH_3$ | 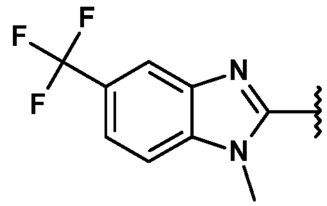 |
| I-39 | $C_2H_5$ | 2 | 8-$SO_2C_2H_5$ | 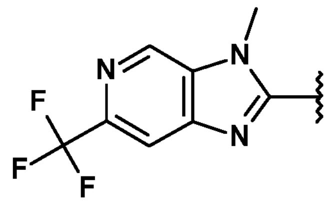 |
| I-40 | $C_2H_5$ | 2 | H | 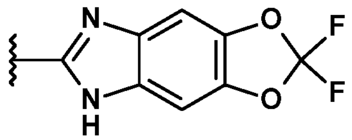 |
| I-41 | $C_2H_5$ | 2 | H | 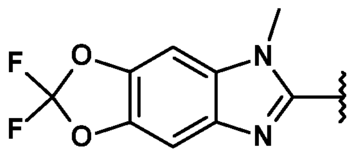 |
| I-42 | $C_2H_5$ | 2 | 6-Cl | 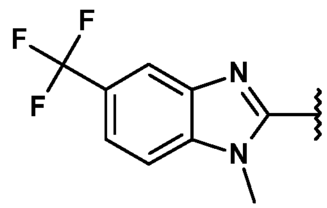 |
| I-45 | $C_2H_5$ | 0 | 6-$CH_3$ | 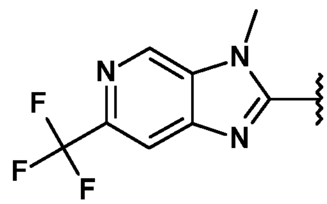 |
| I-46 | $C_2H_5$ | 2 | 5-$CH_3$ | 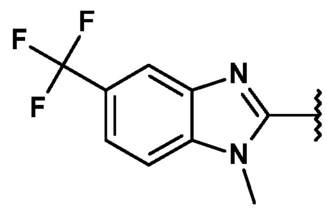 |

(fortgesetzt)

| Bsp. | $R^1$ | n | $R^2$ | Q |
|---|---|---|---|---|
| I-48 | $C_2H_5$ | 2 | 6-$CH_3$ | 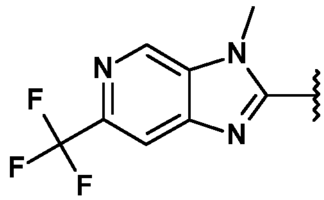 |
| I-54 | $C_2H_5$ | 2 | 6-$OCH_3$ | 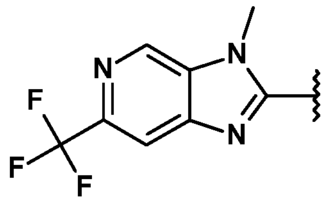 |
| I-55 | $C_2H_5$ | 1 | 6-$OCH_3$ | 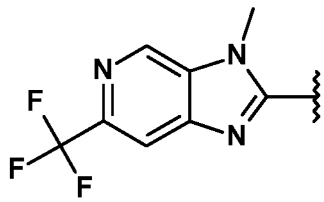 |

<u>**Herstellungsbeispiele für intermediäre Verbindungen:**</u>

**3-(Ethylsulfonyl)imidazo[1,2-a]pyridin-2-carbonsäure (XXIII-1)**

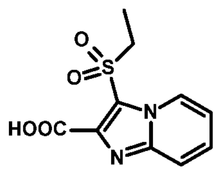

Zu einer Lösung von 161 mg (0,57 mmol) Ethyl-3-(ethylsulfonyl)imidazo[1,2-a]pyridin-2-carboxylat in 5 ml Ethanol wurde 34,1 mg (1,42 mmol) Lithiumhydroxid gegeben und das Gemisch für 3 h bei Raumtemperatur gerührt. Der entstandene Niederschlag wurde gesammelt.
(logP (sauer): 0,54; $MH^+$: 255; $^1$H-NMR(400 MHz, $D_6$-DMSO) $\delta$ ppm: 1,22 (t, 3H), 3,65 (q, 2H), 7,32 (t, 1H), 7,68 (t, 1H), 7,87 (d, 1H), 8,99 (d, 1H).

**Ethyl-3-(ethylsulfonyl)imidazo[1,2-a]pyridin-2-carboxylat (XXIV-1)**

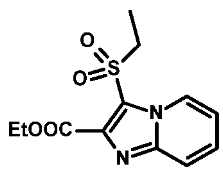

210 mg (0,83 mmol) Ethyl-3-(ethylsulfanyl)imidazo[1,2-a]pyridin-2-carboxylat wurden in 10 ml Dichlormethan gelöst, bei 0 °C 434 mg (2,51 mmol) 3-Chlorbenzolcarboperoxosäure (MCPBA) zugegeben und anschließend 2 h bei

Raumtemperatur gerührt. Der Ansatz wurde mit Wasser verdünnt und mit Natriumbisulfit-Lösung versetzt, 10 min gerührt, und anschließend mit 10%-iger Natriumhydrogencarbonat Lösung gerührt. Die organische Phase wurde abgetrennt, die wässrige Phase zweimal mit Dichlormethan extrahiert und die vereinigten organischen Phasen anschließend unter Vakuum vom Lösungsmittel befreit.
(logP (neutral): 1,52; $MH^+$: 283; $^1H$-NMR(400 MHz, $D_6$-DMSO) $\delta$ ppm: 1,24 (t, 3H), 1,34 (t, 3H), 3,65 (q, 2H), 4,37 (q, 2H), 7,34 (t, 1H), 7,68-7,72 (m, 1H), 7,89 (d, 1H), 8,98 (d, 1H).

**Ethyl-3-(ethylsulfanyl)imidazo[1,2-a]pyridin-2-carboxylat (XXII-1)**

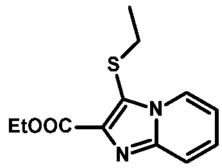

500 mg (2,22 mmol) Ethyl-3-chlorimidazo[1,2-a]pyridin-2-carboxylat wurden in 10 ml N,N-Dimethylformamid gelöst, bei Raumtemperatur 562 mg (6,67 mmol) Natriumthioethanolat zugegeben und anschließend 2,5 h bei Raumtemperatur gerührt. Der Ansatz wurde mit Wasser verdünnt und dreimal mit Essigester extrahiert. Die vereinten organischen Phasen wurden über Natriumsulfat getrocknet und anschließend das Lösungsmittel im Vakuum entfernt. Das Rohprodukt wurde mittels MPLC mit einem Wasser / Acetonitril Gradienten als Laufmittel gereinigt.
(logP (neutral): 2,05; $MH^+$: 251; $^1H$-NMR(400 MHz, $D_6$-DMSO) $\delta$ ppm: 1,06 (t, 3H), 1,35 (t, 3H), 2,88 (q, 2H), 4,34 (q, 2H), 7,15-7,19 (t, 1H), 7,47-7,51 (m, 1H), 7,71 (d, 1H), 8,67 (d, 1H).

**3-Chlor-7-(trifluormethyl)imidazo[1,2-a]pyridin-2-carbonsäure (III-1)**

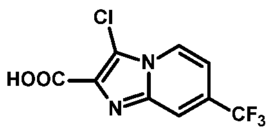

Zu einer Lösung von 1,22 g (4,27 mmol) Ethyl-3-chlor-7-(trifluormethyl)imidazo[1,2-a]pyridin-2-carboxylat in 20 ml Methanol wurde 256 mg (10,6 mmol) Lithiumhydroxid gegeben und das Gemisch über Nacht bei Raumtemperatur gerührt. Das Lösungsmittel wurde entfernt, der Rückstand in Wasser gelöst und die wässrige Phase einmal mit Dichlormethan extrahiert. Anschließend wurde die wässrige Phase mit HCl-Lösung angesäuert und der entstandene Niederschlag gesammelt.
(logP (sauer): 1,50; $MH^+$: 265; $^1H$-NMR(400 MHz, $D_6$-DMSO) $\delta$ ppm: 7,38-7,42 (m, 1H), 8,26 (s, 1H), 8,62 (d, 1H).

**Ethyl-3-chlor-7-(trifluormethyl)imidazo[1,2-a]pyridin-2-carboxylat**

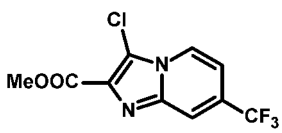

Zu einer Lösung von 136 mg (0,55 mmol) Ethyl-7-(trifluormethyl)imidazo[1,2-a]pyridin-2-carboxylat in 0,6 ml N,N-Dimethylformamid wurde 81,8 mg (0,61 mmol) N-Chlorsuccinimid (NCS) gegeben und das Gemisch für 4 h bei 40 °C gerührt. Die Reaktion wurde mit Natriumhydrogensulfit-Lösung gequenched und die wässrige Phase dreimal mit Essigester extrahiert. Die vereinten organischen Phasen wurden über Natriumsulfat getrocknet und anschließend das Lösungsmittel im Vakuum entfernt.

(logP (neutral): 2,10; $MH^+$: 279; $^1H$-NMR(400 MHz, $D_6$-DMSO) δ ppm: 3,91 (s, 3H), 7,41-7,43 (m, 1H), 8,28 (s, 1H), 8,64 (d, 1H).

**Ethyl-7-(trifluormethyl)imidazo[1,2-a]pyridin-2-carboxylat**

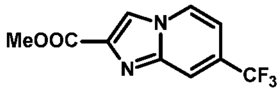

Zu einer Lösung von 5,0 g (30,8 mmol) 4-(Trifluormethyl)pyridin-2-amin in 70 ml Acetonitril wurde 13,8 g (73,9 mmol) Methyl-3-brom-2-oxopropanoat gegeben und das Gemisch für 3 h bei 50 °C gerührt. Der entstandene Niederschlag wurde gesammelt.
(logP (neutral): 1,59; $MH^+$: 245; $^1H$-NMR(400 MHz, $D_6$-DMSO) δ ppm: 3,88 (s, 3H), 7,28-7,31 (m, 1H), 8,16 (s, 1H), 8,74 (s, 1H), 8,79 (d, 1H).

Die Messung der logP Werte erfolgt gemäß EEC Directive 79/831 Annex V.A8 durch HPLC (High Performance Liquid Chromatography) an einer Phasenumkehrsäule (C 18). Temperatur: 55°C.

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. In der Tabelle logP (HCOOH) genannt.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. In der Tabelle logP (neutral) genannt.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Daten ausgewählter Beispiele werden entweder in klassischer Form (δ-Werte, Multiplettaufspaltung, Anzahl der H-Atome) oder als NMR-Peak-Listen aufgeführt.

Das Lösungsmittel, in welchem das NMR-Spektrum aufgenommen wurde ist jeweils angegeben.

NMR-Peak-Listenverfahren

Die $^1H$-NMR-Daten ausgewählter Beispiele werden in Form von $^1H$-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

$$\delta_1 \text{ (Intensität}_1\text{)}; \delta_2 \text{ (Intensität}_2\text{)};\ldots\ldots..; \delta_i \text{ (Intensität}_i\text{)};\ldots\ldots; \delta_n \text{ (Intensität}_n\text{)}$$

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von $^1H$-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der $^1H$-NMR-Peaks sind ähnlich den klassischen $^1H$-NMR-Ausdrucken und enthalten somit gewöhnlich alle Peaks, die bei einer klassischen NMR-Interpretation aufgeführt werden.

Darüber hinaus können sie wie klassische $^1H$-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von $^1H$-NMR-Peaks die gewöhnlichen Lösungsmittelpeaks, zum Beispiel Peaks von DMSO in DMSO-$D_6$ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit

von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen $^1$H-NMR-Interpretation.

Weitere Details zu $^1$H-NMR-Peaklisten können der Research Disclosure Database Number 564025 entnommen werden.

| LOGP_ NEUTRAL | LOGP_ HCOOH | NMR |
|---|---|---|
| 2,05 | 2,08 | Beispiel I-1: $^1$H-NMR(400,0 MHz, $d_6$-DMSO): δ= 9,276(3,9);9,220(0,4);9,083(1,9);9,065 (2,0);8,311(0,4);8,292(4,1);8,265(0,4);8,008(1,7);7,985(2,1);7,787(1,0);7,785(1,1); 7,770(1,2);7,767(1,3);7,747(0,9);7,745(0,9);7,425(1,0);7,423(1,0);7,408(1,9);7,405 (1,9);7,391(0,9);7,388(0,9);4,421(1,8);4,081(16,0);3,902(1,1);3,884(3,5);3,865(3,5); 3,847(1,1 );3,309(74,3);2,674(0,7);2,670(1,0);2,666(0,8);2,523(3,0);2,505(115,6);2,501 (152,9);2,497(116,3);2,332(0,7);2,328(1,0);2,323(0,7);1,299(3,7);1,280(8,1);1,262 (3,6);1,234(0,4);0,008(0,5);0,000(15,0);-0,063(0,4) |
| 2,75 | 2,84 | Beispiel I-2: $^1$H-NMR(400,0 MHz, $d_6$-DMSO): δ= 9,224(4,1);8,823(0,3);8,802(1,8);8,785 (1,8);8,314(0,4);8,261(4,0);7,953(0,7);7,841(1,7);7,818(2,0);7,577(1,0);7,574(1,1); 7,560(1,2);7,557(1,2);7,552(1,0);7,538(1,0);7,535(0,9);7,262(0,3);7,255(1,0);7,239 (1,8);7,237(1,8);7,220(0,9);6,838(0,3);4,507(1,1);4,422(2,0);4,288(16,0);4,019(0,6); 3,386(0,8);3,318(63,2);3,070(1,3);3,05 2(4,1);3,033(4,1);3,015(1,4);2,892(5,3);2,875 (0,4);2,863(0,4);2,812(0,4);2,799(0,4);2,732(4,6);2,676(0,7);2,671(1,0);2,667(0,7); 2,524(2,6);2,507(110,0);2,502(145,5);2,498(110,6);2,333(0,7);2,329(0,9);2,325(0,8); 1,234(0,9);1,221(0,4);1,202(0,6);1,183(0,3);1,093(4,3);1,075(8,9);1,056(4,2);0,146 (1,0);0,008(8,2);0,000(197,0);-0,008(11,7);-0,150(1,0) |
| 2,83 | 2,93 | Beispiel I-7: $^1$H-NMR(400,0 MHz, $d_6$-DMSO): δ= 9,405(2,9);9,402(2,9);9,260(4,2);8,313 (3,0);8,247(4,6);8,014(2,4);7,990(2,8);7,719(1,9);7,714(1,8);7,695(1,5);7,690(1,6); 5,753(2,2);4,443(16,0);4,406(0,6);4,085(0,4);3,777(0,4);3,757(1,1);3,738(1,0);3,723 (1,3);3,705(1,1);3,686(0,4);3,579(0,5);3,561(1,2);3,542(1,3);3,527(1,0);3,508(1,0); 3,490(0,5);3,402(0,3);3,370(0,5);3,315(482,6);2,670(8,1);2,644(0,5);2,505(967,2); 2,501(1216,0);2,422(0,8);2,413(0,8);2,401(0,8);2,395(0,8);2,363(0,5);2,328(7,7);1,412 (3,9);1,393(8,1);1,375(3,6);1,306(0,3);1,260(0,3);1,245(0,3);1,233(0,6);0,000(10,5) |
|  | 3,25 | Beispiel I-8: $^1$H-NMR(400,0 MHz, $d_6$-DMSO): δ= 9,782(1,9);9,275(3,5);9,246(1,6);8,959 (0,9);8,313(0,5);8,284(1,7);8,259(3,6);8,257(3,7);8,152(1,4);8,129(1,7);8,056(0,6); 8,032(0,8);7,884(1,4);7,879(1,4);7,860(1,2);7,855(1,2);7,780(0,7);7,776(0,7);7,756 (0,6);7,752(0,6);4,466(16,0);4,417(7,8);3,788(0,8);3,770(0,9);3,755(1,2);3,737(1,1); 3,603(1,1);3,585(1,2);3,570(0,9);3,551(0,8);3,315(98,3);2,675(0,7);2,671(1,0);2,666 (0,7);2,661(0,3);2,524(2,3);2,519(3,6);2,511(54,7);2,506(114,1);2,502(153,7);2,497 (112,7);2,492(55,0);2,337(0,3);2,333(0,7);2,328(1,0);2,324(0,7);2,073(0,7);1,428(3,6); 1,410(8,1);1,391(3,5);0,146(1,1);0,008(8,5);0,000(245,0);-0,009(9,1);-0,150(1,1) |
| 3,41 | 3,25 | Beispiel I-9: $^1$H-NMR(400,0 MHz, $d_6$-DMSO): δ= 8,786(1,7);8,768(1,8);8,108(2,6);7,902 (1,7);7,880(2,0);7,819(1,7);7,797(2,0);7,675(1,5);7,672(1,4);7,653(1,2);7,651(1,2); 7,554(0,9);7,552(1,0);7,537(1,1);7,534(1,2);7,529(0,8);7,515(0,9);7,512(0,9);7,236 (1,0);7,234(1,0);7,219(1,7);7,217(1,8);7,202(0,9);7,200(0,8);4,149(16,0);3,318(51,5); 3,068(1,2);3,050(4,0);3,031(4,0);3,013(1,3);2,676(0,3);2,671(0,5);2,667(0,4);2,507 (60,2);2,502(78,8);2,498(57,5);2,333(0,4);2,329(0,5);2,324(0,3);1,398(1,4);1,092(4,3); 1,073(8,9);1,055(4,1);0,000(3,9) |

(fortgesetzt)

| LOGP_ NEUTRAL | LOGP_ HCOOH | NMR |
|---|---|---|
| 2,64 | 2,70 | Beispiel I-10: $^1$H-NMR(400,0 MHz, $d_6$-DMSO): δ = 9,095(1,8);9,077(1,9);8,313(0,5);8,125(2,7);7,991(1,7);7,968(2,0);7,945(1,7);7,924(2,0);7,767(1,0);7,747(1,3);7,724(2,2);7,699(1,3);7,403(1,0);7,386(1,7);7,384(1,7);7,369(0,9);3,974(16,0);3,944(1,0);3,926(3,3);3,907(3,4);3,889(1,0);3,317(130,6);2,675(1,0);2,671(1,4);2,666(1,0);2,524(3,6);2,506(179,5);2,501(236,7);2,497(173,9);2,333(1,0);2,328(1,4);2,324(1,1);1,301(3,5);1,283(7,8);1,264(3,4);0,146(0,6);0,008(5,0);0,000(138,8);-0,008(5,3);-0,149(0,7) |
| 2,85 | 2,92 | Beispiel I-11: $^1$H-NMR(400,0 MHz, $d_6$-DMSO): δ = 9,291(1,7);9,274(1,8);8,314(0,4);8,094(2,6);7,930(3,2);7,907(3,4);7,688(1,4);7,685(1,4);7,663(1,2);7,633(0,9);7,630(1,0);7,616(1,1);7,613(1,2);7,607(0,9);7,593(0,9);7,590(0,9);7,243(0,9);7,241(1,0);7,226(1,7);7,224(1,8);7,209(0,9);7,206(0,9);4,329(16,0);3,714(0,8);3,696(1,0);3,681(1,2);3,662(1,2);3,644(0,4);3,559(0,4);3,540(1,1);3,522(1,3);3,507(0,9);3,489(0,8);3,317(96,6);2,675(0,9);2,671(1,2);2,666(0,9);2,524(2,9);2,510(73,1);2,506(152,3);2,501(204,3);2,497(149,3);2,493(73,0);2,333(0,8);2,328(1,2);2,324(0,9);1,388(3,8);1,369(8,3);1,351(3,7);0,146(0,4);0,008(2,9);0,000(88,7);-0,008(3,4);-0,150(0,4) |
| 3,71 | 1,40 | Beispiel I-13: $^1$H-NMR(400,0 MHz, $d_6$-DMSO): δ = 9,231(4,1);9,061(1,8);9,044(1,9);8,288(4,5);8,047(1,7);8,030(1,8);7,381(1,1);7,363(2,1);7,346(1,1);4,287(16,0);3,331(187,0);3,325(341,4);3,102(1,3);3,083(4,0);3,065(4,1);3,047(1,4);2,671(1,2);2,506(159,0);2,502(194,2);2,498(142,7);2,329(1,2);1,398(1,2);1,235(0,6);1,111(4,2);1,093(8,7);1,074(4,1);0,000(4,6);-0,086(0,5) |
| 3,34 | 3,33 | Beispiel I-14: $^1$H-NMR(400,0 MHz, $d_6$-DMSO): δ = 9,255(0,4);9,233(3,3);8,796(1,7);8,794(1,8);8,779(1,9);8,777(1,9);8,313(0,7);8,276(3,5);7,779(1,7);7,777(1,9);7,760(1,9);7,758(1,9);7,245(1,5);7,227(2,4);7,209(1,5);4,318(2,1);4,302(16,0);3,374(0,5);3,321(208,6);3,083(1,2);3,065(3,8);3,047(3,9);3,028(1,2);2,679(0,7);2,675(1,4);2,671(2,0);2,666(1,5);2,524(5,2);2,510(119,9);2,506(252,7);2,502(340,5);2,497(244,4);2,492(115,6);2,333(1,4);2,328(1,9);2,324(1,4);2,319(0,7);1,236(0,5);1,097(4,3); 1,079(8,9);1,060(4,1);0,146(0,9);0,008(7,1);0,000(211,9);-0,009(7,6);-0,150(0,9) |
| 3,93 | 3,99 | Beispiel I-15: $^1$H-NMR(400,0 MHz, $d_6$-DMSO): δ = 9,233(0,5);9,215(3,1);8,592(1,7);8,577(1,7);8,575(1,7);8,314(0,5);8,275(0,5);8,260(3,3);7,395(1,5);7,379(1,7);7,227(0,3);7,207(1,5);7,190(2,2);7,172(1,2);4,302(16,0);3,316(96,3);3,244(1,0);3,226(3,2);3,208(3,3);3,189(1,0);3,070(1,1);3,066(0,8);3,052(3,6);3,033(3,7);3,015(1,1);2,675(0,9);2,671(1,2);2,666(0,9);2,524(2,6);2,519(4,1);2,510(71,3);2,506(153,0);2,501(207,6);2,497(150,8);2,493(72,9);2,333(0,9);2,328(1,2);2,324(0,9);1,375(3,5);1,356(7,7);1,338(3,4);1,098(0,7);1,091(3,9);1,079(1,5);1,072(8,4);1,061(0,8);1,054(3,8);0,146(1,1);0,008(8,4);0,000(270,2);-0,009(10,6);-0,150(1,2) |
| 3,25 | 3,29 | Beispiel I-18: $^1$H-NMR(400,0 MHz, $d_6$-DMSO): δ = 9,507(1,6);9,489(1,5);9,273(3,6);8,475(2,1);8,312(0,4);8,266(3,7);7,527(1,4);7,522(1,4);7,508(1,3);7,503(1,3);4,457(16,0);3,743(0,9);3,724(0,9);3,709(1,2);3,691(1,2);3,672(0,4);3,577(1,2);3,559(1,3);3,544(0,9);3,525(0,8);3,508(0,3);3,399(0,3);3,329(149,5);3,323(124,3);2,675(0,9);2,671(1,2);2,524(3,0);2,511(70,7);2,507(147,2);2,502(197,0);2,498(141,0);2,493(66,8);2,333(0,8);2,329(1,1);2,324(0,8);1,411(3,6);1,392(8,0);1,374(3,6);0,146(0,4);0,008(2,3);0,000(70,4);-0,009(2,4) |
| 2,50 | 2,56 | Beispiel I-19: $^1$H-NMR(400,0 MHz, $d_6$-DMSO): δ = 9,279(4,0);9,045(2,1);9,044(2,1);9,028(2,3);9,026(2,1);8,307(4,3);7,990(2,2);7,971(2,4);7,403(1,6);7,385(2,6);7,367(1,6);5,753(1,1);4,084(16,0);3,894(1,0);3,875(3,4);3,857(3,5);3,839(1,1);3,317(87,8);2,675(0,6);2,671(0,8);2,666(0,6);2,506(103,4);2,502(134,9);2,497(98,8);2,329(0,8);1,299(3,6);1,281(8,0);1,263(3,6);1,234(0,5);0,146(0,7);0,008(6,5);0,000(153,0);-0,008(6,9);-0,150(0,7) |

(fortgesetzt)

| LOGP_ NEUTRAL | LOGP_ HCOOH | NMR |
|---|---|---|
| | 3,50 | Beispiel I-20: $^1$H-NMR(400,0 MHz, $d_6$-DMSO): δ = 9,256(3,5);8,313(0,5);8,300(3,8);8,176 (1,5);8,154(1,6);7,898(1,4);7,881(1,6);7,669(1,0);7,648(1,2);7,629(0,9);4,139(16,0); 3,373(0,5);3,362(0,5);3,352(0,7);3,342(1,3);3,320(261,2);3,293(0,6);2,963(0,9);2,945 (2,9);2,926(3,0);2,907(1,0);2,675(1,0);2,671(1,4);2,666(1,0);2,524(3,1);2,511(80,7); 2,506(170,3);2,502(229,0);2,497(165,4);2,493(79,0);2,337(0,5);2,333(1,0);2,328(1,3); 2,324(0,9);1,045(0,4);1,030(0,4);1,008(3,6);0,989(7,7);0,971(3,5);0,146(0,6);0,023 (0,4);0,008(4,7);0,000(141,9);-0,008(4,4);-0,019(0,6);-0,150(0,6) |
| 2,66 | 2,69 | Beispiel I-21: $^1$H-NMR(400,0 MHz, $d_6$-DMSO): δ = 9,292(4,3);8,335(1,9);8,308(5,3);8,095 (1,8);8,076(2,2);7,932(1,3);7,910(1,6);7,890(1,0);4,080(16,0);3,879(0,8);3,861(2,6); 3,842(2,6);3,823(0,9);3,335(217,4);3,329(279,2);2,671(1,3);2,506(172,3);2,502(227,1); 2,498(172,5);2,329(1,3);2,073(0,4);1,315(3,4);1,297(7,4);1,278(3,4);0,008(0,8);0,000 (25,0) |
| 2,30 | 2,30 | Beispiel I-22: $^1$H-NMR(400,0 MHz, $d_6$-DMSO): δ = 9,251(3,6);8,311(4,2);8,265(1,5);8,242 (1,6);7,972(1,4);7,955(1,7);7,771(1,1);7,750(1,2);7,731(0,9);4,156(0,6);4,137(0,7); 4,123(0,8);4,104(0,6);3,861(16,0);3,773(0,4);3,371(0,6);3,354(0,8);3,317(236,7);3,284 (0,4);2,675(1,1);2,671(1,6);2,666(1,2);2,524(3,8);2,510(92,6);2,506(189,6);2,501 (251,2);2,497(183,2);2,492(90,7);2,337(0,5);2,333(1,1);2,328(1,5);2,324(1,1);2,319 (0,5);1,287(2,8);1,268(6,2);1,249(2,8);0,008(2,1);0,000(63,0);-0,008(2,7) |
| 2,90 | 2,92 | Beispiel I-24: $^1$H-NMR(400,0 MHz, $d_6$-DMSO): δ = 9,290(2,1);9,276(6,1);8,314(5,0);8,250 (1,9);8,231(2,0);7,544(1,2);7,525(2,2);7,507(1,1);4,083(16,0);3,941(1,1);3,923(3,6); 3,904(3,7);3,886(1,2);3,323(397,4);2,671(1,6);2,505(229,6);2,502(274,8);2,328(1,5); 1,325(3,8);1,306(8,1);1,288(3,7);0,000(19,3) |
| 3,21 | 3,29 | Beispiel I-25: $^1$H-NMR(400,0 MHz, $d_6$-DMSO): δ = 9,539(1,9);9,522(2,0);9,254(4,3);8,265 (4,6);8,122(1,8);8,104(1,9);7,399(1,1);7,381(2,2);7,363(1,1);4,448(16,0);3,736(0,8); 3,718(1,0);3,703(1,3);3,684(1,2);3,666(0,4);3,591(0,4);3,573(1,2);3,554(1,3);3,539 (1,0);3,521(0,8);3,324(241,3);2,676(0,6);2,671(0,7);2,507(101,0);2,502(127,1);2,498 (96,3);2,329(0,7);1,412(3,9);1,393(8,3);1,374(3,8);0,000(10,6) |
| | 3,35 | Beispiel I-26: $^1$H-NMR(400,0 MHz, $d_6$-DMSO): δ = 8,111(4,3);7,908(2,1);7,887(2,5);7,803 (0,4);7,685(2,7);7,672(2,5);7,665(2,5);7,650(2,5);7,433(1,5);7,415(2,0);7,394(1,4); 7,225(0,4);6,941(2,3);6,924(2,1);4,155(2,1);3,999(16,0);3,908(2,0);3,336(185,7);3,172 (14,9);3,055(0,7);3,037(0,7);2,993(1,6);2,975(4,9);2,957(5,0);2,938(1,6);2,678(0,8); 2,509(146,0);2,505(111,6);2,335(0,8);1,097(0,8);1,079(1,6);1,062(6,1);1,044(11,6); 1,025(5,4) |
| | 4,09 | Beispiel I-27: $^1$H-NMR(400,0 MHz, $d_6$-DMSO): δ = 8,165(1,9);8,143(5,2);7,935(1,8);7,914 (2,2);7,884(1,9);7,866(2,2);7,711(1,8);7,690(1,5);7,655(1,3);7,635(1,5);7,615(1,0); 4,107(0,4);4,094(0,4);4,013(16,0);3,908(2,6);3,337(150,3);3,182(2,5);3,169(2,4);2,974 (1,1);2,956(3,4);2,937(3,4);2,919(1,1);2,682(0,5);2,678(0,6);2,513(85,0);2,509(111,8); 2,505(86,7);2,336(0,6);1,015(4,0);0,997(8,3);0,978(3,9) |
| | 4,47 | Beispiel I-28: $^1$H-NMR(400,0 MHz, $d_6$-DMSO): δ = 9,082(2,7);8,140(3,5);8,046(1,9);8,022 (2,3);7,932(1,8);7,911(2,1);7,795(1,8);7,791(1,8);7,772(1,5);7,768(1,5);7,703(2,0); 7,681(1,6);7,679(1,6);4,167(16,0);3,909(1,6);3,337(159,2);3,137(1,4);3,119(4,5);3,101 (4,6);3,082(1,5);2,679(0,6);2,514(80,2);2,510(107,6);2,505(84,3);2,336(0,6);1,114 (4,8);1,096(10,0);1,077(4,7) |
| | 4,12 | Beispiel I-29: $^1$H-NMR(400,0 MHz, $d_6$-DMSO): δ = 8,979(1,9);8,961(2,0);8,873(1,0);8,349 (3,0);8,140(3,4);8,120(1,2);7,930(1,8);7,909(2,4);7,893(0,8);7,884(0,9);7,860(0,9); 7,702(2,0);7,682(2,1);7,665(0,6);7,605(0,6);7,602(0,6);7,581(0,5);7,467(1,8);7,449 (1,7);4,166(16,0);4,150(5,0);3,908(1,9);3,338(175,6);3,129(1,4);3,111(4,4);3,092(4,5); 3,079(1,9);3,075(1,7);3,061(1,5);3,042(0,5);2,678(0,7);2,513(89,3);2,509(114,2);2,505 (88,1);2,336(0,6);1,111(5,9);1,093(12,1);1,074(5,7) |

(fortgesetzt)

| LOGP_ NEUTRAL | LOGP_ HCOOH | NMR |
|---|---|---|
| | 4,50 | Beispiel I-30: $^1$H-NMR(400,0 MHz, $d_6$-DMSO): δ = 8,980(1,9);8,962(2,0);8,873(1,7);8,350(3,0);8,140(3,4);8,120(2,0);7,930(1,9);7,910(2,6);7,892(1,3);7,885(1,5);7,861(1,5);7,702(2,0);7,683(2,5);7,665(1,0);7,607(1,0);7,602(1,0);7,583(0,9);7,579(0,8);7,469(1,7);7,465(1,7);7,451(1,7);7,447(1,7);4,166(16,0);4,150(8,2);3,908(2,9);3,338(300,3);3,130(1,4);3,111(4,2);3,093(4,5);3,079(2,7);3,075(2,0);3,061(2,4);3,043(0,8);2,678(1,0);2,513(134,3);2,509(176,3);2,505(139,6);2,336(1,0);1,111(6,2);1,093(12,4);1,075(5,9) |
| | 3,95 | Beispiel I-31: $^1$H-NMR(400,0 MHz, $d_6$-DMSO): δ = 8,116(4,9);7,909(2,1);7,888(2,5);7,686(3,0);7,665(2,5);7,607(2,6);7,585(3,5);7,484(1,9);7,465(2,7);7,444(1,5);7,310(1,3);7,289(2,6);7,267(2,6);7,245(1,0);7,067(3,4);7,049(3,0);4,150(0,4);4,005(16,0);3,908(4,6);3,341(289,7);3,228(1,7);3,210(5,1);3,191(5,2);3,173(1,8);3,005(1,7);2,987(5,2);2,968(5,3);2,950(1,8);2,678(0,9);2,571(0,4);2,509(159,2);2,335(0,9);1,657(3,1);1,406(5,6);1,387(11,5);1,369(5,4);1,264(0,4);1,239(0,7);1 ,065(6,2);1,047(12,4);1,028(5,8) |
| | 4,23 | Beispiel I-32: $^1$H-NMR(400,0 MHz, $d_6$-DMSO): δ = 8,630(2,6);8,612(2,7);8,320(0,4);8,105(5,6);7,898(2,2);7,877(2,5);7,675(3,6);7,653(2,9);7,572(4,5);7,089(3,3);7,086(3,2);7,071(3,2);7,068(3,1);4,151(15,5);3,908(6,8);3,516(0,4);3,458(0,4);3,337(473,1);3,214(2,4);3,196(7,3);3,178(7,5);3,159(2,4);3,060(2,0);3,041(6,2);3,023(6,3);3,005(2,2);2,678(1,9);2,513(252,5);2,509(333,0);2,505(259,0);2,336(1,8);1,367(7,8);1,349(16,0);1,331(7,5);1,305(0,3);1,256(0,5);1,239(0,5);1,097(7,6);1,079(15,4);1,060(7,3);0,006(0,6) |
| | 3,01 | Beispiel I-33: $^1$H-NMR(400,0 MHz, $d_6$-DMSO): δ = 8,970(2,1);8,952(2,1);8,123(2,8);7,943(1,7);7,922(2,1);7,776(2,6);7,724(1,6);7,721(1,6);7,703(1,3);7,700(1,3);7,258(1,5);7,254(1,5);7,240(1,5);7,236(1,5);3,969(16,0);3,922(1,0);3,909(2,9);3,903(3,2);3,885(3,2);3,867(1,0);3,337(104,5);2,683(0,4);2,678(0,5);2,514(58,2);2,509(78,4);2,505(61,2);2,500(37,7);2,336(0,5);1,291(3,4);1,273(7,6);1,254(3,3) |
| | 3,72 | Beispiel I-34: $^1$H-NMR(400,0 MHz, $d_6$-DMSO): δ = 9,272(1,8);9,253(1,9);8,550(2,6);8,157(3,0);7,964(1,7);7,943(2,1);7,748(1,7);7,727(1,4);7,665(1,5);7,647(1,5);4,018(1,1);3,998(16,0);3,982(3,2);3,964(1,0);3,908(1,6);3,336(143,8);2,678(0,6);2,509(106,9);2,335(0,6);1,332(3,2);1,314(6,8);1,296(3,1) |
| | 2,98 | Beispiel I-35: $^1$H-NMR(400,0 MHz, $d_6$-DMSO): δ = 8,867(2,8);8,121(3,1);7,941(1,8);7,919(2,3);7,910(2,2);7,887(2,4);7,721(1,7);7,700(1,4);7,639(1,7);7,636(1,8);7,616(1,5);7,613(1,5);3,954(16,0);3,918(1,0);3,908(2,1);3,900(3,4);3,881(3,3);3,863(1,0);3,336(88,8);2,678(0,4);2,513(51,9);2,509(68,8);2,505(55,0);2,454(10,4);2,336(0,4);1,305(3,5);1,287(7,7);1,268(3,4) |
| | 3,70 | Beispiel I-36: $^1$H-NMR(400,0 MHz, $d_6$-DMSO): δ = 9,422(3,3);8,213(2,0);8,189(2,5);8,160(3,8);8,031(2,2);8,007(1,7);7,967(2,1);7,945(2,5);7,749(2,1);7,727(1,7);4,088(1,2);4,070(3,5);4,051(3,6);4,033(1,3);4,005(16,0);3,908(2,0);3,335(209,2);2,678(0,9);2,509(145,5);2,335(0,8);1,352(3,8);1,333(7,9);1,315(3,7) |
| | 3,41 | Beispiel I-37: $^1$H-NMR(400,0 MHz, $d_6$-DMSO): δ = 8,664(2,3);8,647(2,3);8,100(4,9);7,896(1,9);7,875(2,2);7,670(3,2);7,649(2,6);7,587(4,0);7,075(3,0);7,058(2,9);4,139(13,9);3,908(1,9);3,339(213,0);3,183(0,4);3,170(0,4);3,047(1,7);3,029(5,0);3,010(5,0);2,992(1,8);2,679(0,8);2,509(125,8);2,457(16,0);2,336(0,7);1,084(5,8);1,066(11,7);1,047(5,7) |
| | 3,57 | Beispiel I-38: $^1$H-NMR(400,0 MHz, $d_6$-DMSO): δ = 8,578(2,9);8,102(4,1);7,898(1,6);7,877(1,9);7,733(2,3);7,710(2,6);7,672(2,6);7,651(2,1);7,410(2,1);7,387(1,8);4,142(12,6);3,908(2,4);3,342(304,3);3,061(1,6);3,042(4,8);3,024(4,9);3,006(1,6);2,679(0,8);2,675(0,7);2,514(108,0);2,510(145,7);2,505(113,6);2,436(16,0);2,336(0,8);1,095(5,9);1,077(12,2);1,058(5,8) |

(fortgesetzt)

| LOGP_ NEUTRAL | LOGP_ HCOOH | NMR |
|---|---|---|
| 2,29 | 2,30 | Beispiel I-39: $^1$H-NMR(400,0 MHz, $d_6$-DMSO): δ = 9,343(2,2);9,325(2,2);9,290(4,5);9,279 (0,6);8,322(4,7);8,306(2,8);8,287(2,3);7,605(1,7);7,587(3,1);7,569(1,6);4,105(16,0); 4,083(1,9);3,982(1,0);3,963(3,5);3,945(3,6);3,926(1,1);3,875(0,4);3,857(0,4);3,792 (1,0);3,774(3,4);3,755(3,5);3,737(1,1);3,319(170,9);2,675(0,6);2,671(0,8);2,667(0,6); 2,506(102,2);2,502(137,0);2,497(105,1);2,333(0,6);2,329(0,8);2,324(0,6);2,073(1,5); 1,353(3,7);1,335(8,1);1,316(3,6);1,299(0,5);1,281(0,9);1,262(0,4);1,211(3,7);1,193 (8,0);1,174(3,6);0,146(0,6);0,000(115,2);-0,008(5,8);-0,150(0,6) |
| 2,59 | 2,51 | Beispiel I-40: $^1$H-NMR(400,0 MHz, $d_6$-DMSO): δ= 19,785(0,6);18,114(0,6);13,547(5,4); 10,632(1,0);9,399(0,5);9,227(4,7);9,209(5,2);9,138(0,6);8,314(1,9);7,915(4,3);7,892 (5,2);7,865(0,6);7,840(0,7);7,768(11,2);7,721(2,8);7,704(3,8);7,684(2,7);7,526(11,2); 7,353(0,7);7,343(2,9);7,325(4,8);7,308(2,6);4,201(2,3);4,183(7,0);4,165(7,1);4,147 (2,3);3,821(0,6);3,818(0,6);3,801(0,9);3,781(0,8);3,503(0,5);3,456(0,6);3,414(0,7); 3,398(0,9);3,317(812,1);3,024(0,6);2,897(0,5);2,754(0,6);2,741(0,9);2,726(0,8);2,670 (8,9);2,620(1,3);2,504(1263,4);2,501(1446,2);2,328(8,0);1,294(7,8);1,275(16,0);1,257 (8,4);1,235(3,1);1,192(1,1);1,175(1,8);1,155(1,1);0,856(0,7);0,146(5,2);0,117(0,6); 0,003(638,7);0,000(1099,3);-0,009(118,6);-0,150(5,2);-3,158(0,5) |
| 2,62 | 2,59 | Beispiel I-41: $^1$H-NMR(601,6 MHz, CD3CN): δ= 9,118(0,9);9,117(1,5);9,115(0,9);9,107 (0,9);9,105(1,5);9,103(0,9);7,803(0,8);7,801(1,4);7,788(1,0);7,786(1,6);7,635(0,8); 7,633(0,9);7,624(0,9);7,622(1,1);7,621(0,9);7,618(0,8);7,609(0,8);7,607(0,8);7,500 (3,8);7,423(4,0);7,230(0,8);7,228(0,8);7,218(1,4);7,216(1,4);7,206(0,7);7,204(0,7); 5,444(1,5);3,917(16,0);3,842(1,1);3,830(3,5);3,818(3,6);3,806(1,2);2,118(25,1);1,962 (0,9);1,954(2,3);1,950(3,0);1,946(15,0);1,942(25,6);1,938(37,5);1,933(25,8);1,929 (13,1);1,341(0,5);1,333(3,7);1,321(7,7);1,308(3,7);1,285(0,7);1,270(0,6);0,000(4,6) |
| | 3,34 | Beispiel I-42: $^1$H-NMR(400,0 MHz, $d_6$-DMSO): δ = 9,115(2,6);9,112(2,6);8,140(2,7);8,060 (1,9);8,036(2,4);7,953(1,6);7,931(2,0);7,852(1,7);7,847(1,6);7,828(1,4);7,823(1,3); 7,736(1,5);7,714(1,2);4,021(0,9);4,002(3,1);3,986(16,0);3,966(1,0);3,908(1,9);3,339 (121,9);2,678(0,5);2,674(0,4);2,513(66,4);2,509(86,8);2,505(66,0);2,340(0,4);2,336 (0,5);2,332(0,4);1,336(3,1);1,318(6,9);1,299(3,1) |
| 3,22 | 3,20 | Beispiel I-45: $^1$H-NMR(400,0 MHz, $d_6$-DMSO): δ = 9,212(3,8);8,587(2,3);8,314(0,5);8,249 (4,1);7,750(1,9);7,727(2,1);7,430(1,5);7,426(1,5);7,407(1,3);7,403(1,4);6,837(0,4); 4,276(16,0);3,316(184,0);3,058(1,3);3,040(3,9);3,022(4,0);3,003(1,3);2,811(0,5);2,798 (0,5);2,675(1,2);2,671(1,6);2,666(1,2);2,524(3,4);2,510(94,0);2,506(199,6);2,501 (280,0);2,497(209,5);2,434(9,2);2,366(0,9);2,333(1,3);2,328(1,7);2,324(1,3);1,458 (0,3);1,138(0,3);1,128(0,3);1,091(4,2);1,072(8,9);1,054(4,1);0,146(0,9);0,008(6,1); 0,000(194,1);-0,008(6,6);-0,150(0,9) |
| | 2,73 | Beispiel I-46: $^1$H-NMR(400,0 MHz, $d_6$-DMSO): δ = 8,092(3,0);7,924(1,8);7,902(2,2);7,861 (1,5);7,839(2,0);7,733(1,5);7,715(1,9);7,707(2,0);7,693(1,4);7,685(1,4);7,273(1,7); 7,256(1,6);3,909(2,2);3,836(16,0);3,824(3,7);3,805(3,4);3,787(1,1);3,335(97,7);3,051 (11,2);2,678(0,7);2,674(0,5);2,513(82,6);2,509(109,3);2,505(84,8);2,336(0,6);1,347 (3,7);1,329(8,1);1,310(3,6) |
| 2,36 | 2,43 | Beispiel I-48: $^1$H-NMR(400,0 MHz, $d_6$-DMSO): δ = 9,267(3,9);8,849(2,4);8,284(4,1);7,922 (1,9);7,899(2,2);7,654(1,6);7,651(1,6);7,631(1,4);7,628(1,4);4,056(16,0);3,876(1,0); 3,857(3,3);3,839(3,3);3,820(1,0);3,319(146,9);2,675(0,5);2,671(0,7);2,666(0,5);2,524 (1,6);2,511(37,9);2,506(78,9);2,502(109,4);2,497(81,1);2,493(38,6);2,456(9,2);2,333 (0,5);2,329(0,6);2,324(0,5);2,073(0,6);1,296(3,5);1,277(7,7);1,259(3,4);0,146(0,3); 0,008(2,6);0,000(79,4);-0,008(2,6);-0,150(0,4) |

(fortgesetzt)

| LOGP_ NEUTRAL | LOGP_ HCOOH | NMR |
|---|---|---|
| 2,37 | 2,41 | Beispiel I-54: $^1$H-NMR(400,0 MHz, $d_6$-DMSO): $\delta$ = 9,265(3,8);8,582(2,4);8,576(2,4);8,314 (0,5);8,283(4,1);7,948(2,3);7,923(2,6);7,587(1,9);7,581(1,9);7,563(1,6);7,557(1,7); 4,425(0,4);4,069(16,0);3,917(16,0);3,903(3,4);3,885(3,2);3,866(1,3);3,315(56,6);2,675 (0,9);2,670(1,3);2,666(1,0);2,524(3,5);2,510(71,9);2,506(152,7);2,501(215,5);2,497 (165,6);2,492(83,1);2,333(0,9);2,328(1,2);2,324(1,0);1,988(0,3);1,308(3,3);1,290(7,7); 1,271(3,3);0,008(2,2);0,000(65,1);-0,008(2,7) |
| 2,48 | 2,54 | Beispiel I-55: $^1$H-NMR(400,0 MHz, $d_6$-DMSO): $\delta$ = 9,575(0,4);9,235(4,7);9,145(0,6);8,869 (2,7);8,863(2,7);8,607(0,6);8,314(1,4);8,222(5,0);8,183(0,7);7,888(2,7);7,863(3,0); 7,454(1,7);7,448(1,7);7,429(1,6);7,423(1,6);4,425(16,0);4,070(0,8);4,018(2,3);3,917 (1,2);3,901(0,9);3,867(15,8);3,848(0,4);3,804(0,8);3,736(0,5);3,718(1,0);3,699(1,0); 3,685(1,5);3,666(1,3);3,648(0,6);3,565(0,5);3,547(1,3);3,528(1,6);3,513(0,9);3,495 (1,0);3,487(0,5);3,476(0,6);3,380(0,5);3,317(383,6);2,890(0,4);2,743(0,4);2,671(5,4); 2,655(0,7);2,650(0,6);2,630(1,1);2,574(1,0);2,556(1,3);2,505(702,5);2,501(918,0); 2,498(711,7);2,409(0,4);2,347(0,6);2,328(5,4);1,395(3,9);1,376(8,1);1,358(3,8);1,236 (0,9);1,179(0,6);1,161(0,6);0,147(1,2);0,000(216,8);-0,149(1,1) |

## Anwendungsbeispiele

### Ctenocephalides felis-in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis*, wenn in diesem Test bei einer Aufwandmenge von 5 $\mu g/cm^2$ mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 $\mu g/cm^2$ (= 500 g/ha): I-1, I-18, I-33, I-34, I-35,

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt. Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus*, wenn in diesem Test bei einer Aufwandmenge von 5 $\mu g/cm^2$ mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 80 % bei einer Aufwandmenge von 5 μg/cm$^2$ (= 500 g/ha): I-35

**Boophilus microplus -Injektionstest**

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.
1μl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.
Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.
Bei diesem Test zeigen z.B. die folgenden Verbindungen der Herstellungsbeispiele eine Wirkung von 95 % bei einer Aufwandmenge von 20 μg/Tier: I-18

**Ctenocephalides felis - Oraltest**

Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.
Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.
Nach 2 Tagen wird die Abtötung in % bestimmt. Dabei bedeutet 100 %, dass alle Flöhe abgetötet wurden; 0 % bedeutet, dass keiner der Flöhe abgetötet wurde.
Bei diesem Test zeigen z.B. die folgenden Verbindungen der Herstellungsbeispiele eine Wirkung von 100% bei einer Aufwandmenge von 100 ppm: I-18
Bei diesem Test zeigen z.B. die folgenden Verbindungen der Herstellungsbeispiele eine Wirkung von 98 % bei einer Aufwandmenge von 100 ppm: I-1

**Lucilia cuprina - Test**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.
Ca. 20 L1-Larven der AustRhipiceralischen Schafgoldfliege (*Lucilia cuprina*) werden in ein Testgefäß überführt, welches gehacktes Pferdefleisch und die Wirkstoffzubereitung der gewünschten Konzentration enthält.
Nach 2 Tagen wird die Abtötung in % bestimmt. Dabei bedeutet 100 %, dass alle Larven abgetötet wurden; 0 % bedeutet, dass keine Larven abgetötet wurden.
Bei diesem Test zeigen z.B. die folgenden Verbindungen der Herstellungsbeispiele eine Wirkung von 100% bei einer Aufwandmenge von 100 ppm: I-1, I-10, I-18

**Musca domestica-Test**

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.
Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.
Nach 2 Tagen wird die Abtötung in % bestimmt. Dabei bedeutet 100 %, dass alle Fliegen abgetötet wurden; 0 % bedeutet, dass keine der Fliegen abgetötet wurde.
Bei diesem Test zeigen z.B. die folgenden Verbindungen der Herstellungsbeispiele eine Wirkung von 95% bei einer Aufwandmenge von 4 ppm: I-18
Bei diesem Test zeigen z.B. die folgenden Verbindungen der Herstellungsbeispiele eine Wirkung von 85% bei einer Aufwandmenge von 4 ppm: I-1

**Myzus persicae - Sprühtest**

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.
Nach 5-6 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 100% bei einer Aufwandmenge von 500 g/ha: I-21
Bei diesem Test zeigen z. B. die folgenden Verbindungen der Herstellungsbeispiele Wirkung von 90% bei einer Aufwandmenge von 500 g/ha: I-1, I-2, I-7, I-11, I-19, I-22, I-35, I-41, I-46, I-48, I-54

**Phaedon cochleariae - Sprühtest**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.
Nach 7 Tagen wird die Wirkung in % bestimmt. Dabei bedeutet 100 %, dass alle Käferlarven abgetötet wurden; 0 % bedeutet, dass keine Käferlarven abgetötet wurden.
Bei diesem Test zeigen z. B. die folgenden Verbindungen der Herstellungsbeispiele Wirkung von 100% bei einer Aufwandmenge von 500 g/ha: I-8, I-9, I-10, I-11, I-13, I-18, I-19, I-21, I-22, I-24, I-25, I-29, I-34, I-36, I-39, I-40, I-41, I-42,
**[0339]** Bei diesem Test zeigen z. B. die folgenden Verbindungen der Herstellungsbeispiele Wirkung von 83% bei einer Aufwandmenge von 500 g/ha: I-1, I-26, I-30, I-46

**Spodoptera frugiperda - Sprühtest**

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 500 g/ha: I-1, I-7, I-8, I-10, I-11, I-13, I-18, I-19, I-21, I-24, I-25, I-28, I-29, I-30, I-34, I-35, I-36, I-39, I-42, I-48, I-54

Bei diesem Test zeigen z. B. die folgenden Verbindungen der Herstellungsbeispiele Wirkung von 83% bei einer Aufwandmenge von 500 g/ha: I-33, I-41

**Myzus persicae - Sprühtest**

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 100 % bei einer Aufwandmenge von 100 ppm: I-10

## Patentansprüche

**1.** Verbindungen der Formel (I)

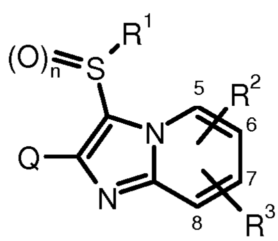

**(I)**

wobei $R^3$ für Wasserstoff steht und wobei $R^1$, $R^2$, Q und n, die in der folgenden Tabelle angegebenen Bedeutungen

haben und wobei die Bindung von Q zum Restmolekül mit einer Wellenlinie gekennzeichnet ist:

| | $R^1$ | n | $R^2$ | Q |
|---|---|---|---|---|
| I-1 | $C_2H_5$ | 2 | H | 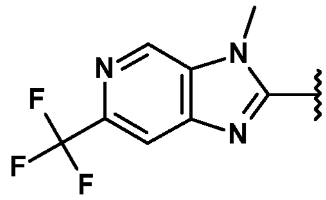 |
| I-2 | $C_2H_5$ | 0 | H | 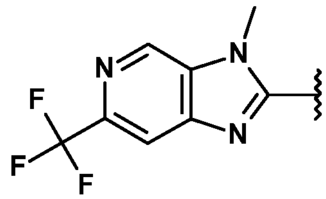 |
| I-7 | $C_2H_5$ | 1 | 6-Cl | 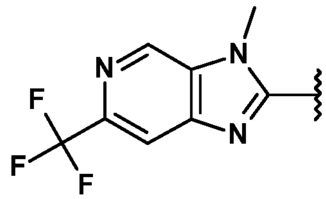 |
| I-8 | $C_2H_5$ | 1 | 6-$CF_3$ | 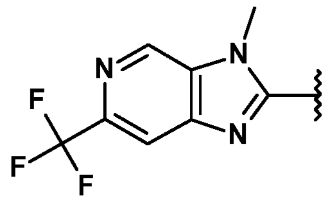 |
| I-9 | $C_2H_5$ | 0 | H | 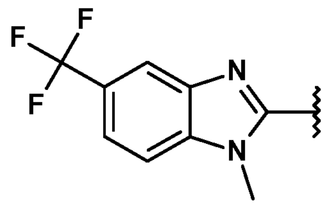 |
| I-10 | $C_2H_5$ | 2 | H | 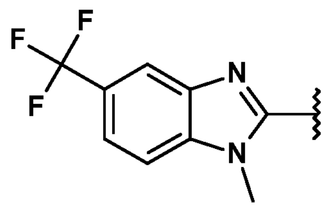 |
| I-11 | $C_2H_5$ | 1 | H | 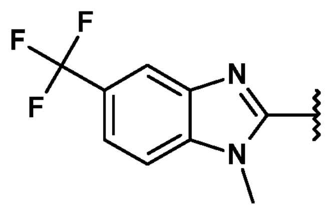 |

(fortgesetzt)

| | $R^1$ | n | $R^2$ | Q |
|---|---|---|---|---|
| I-13 | $C_2H_5$ | 0 | 8-$CF_3$ | 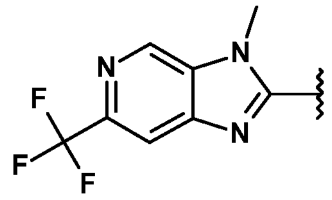 |
| I-14 | $C_2H_5$ | 0 | 8-Cl | 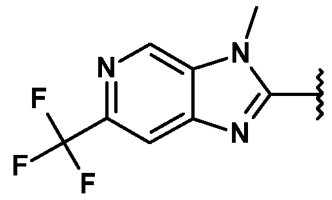 |
| I-15 | $C_2H_5$ | 0 | 8-$SC_2H_5$ | 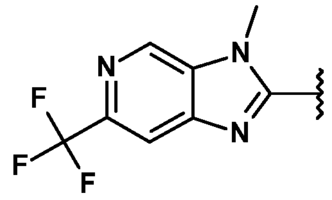 |
| I-18 | $C_2H_5$ | 1 | 7-$CF_3$ | 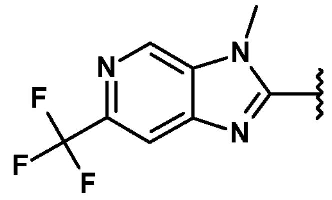 |
| I-19 | $C_2H_5$ | 2 | 8-Cl | 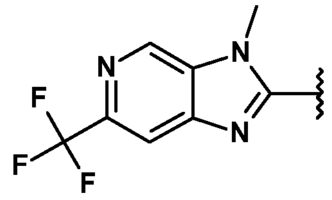 |
| I-20 | $C_2H_5$ | 0 | 5-$CF_3$ | 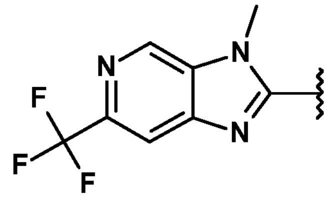 |
| I-21 | $C_2H_5$ | 2 | 5-$CF_3$ | 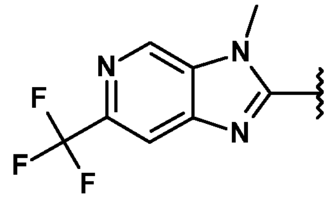 |

(fortgesetzt)

| | $R^1$ | n | $R^2$ | Q |
|---|---|---|---|---|
| I-22 | $C_2H_5$ | 1 | 5-$CF_3$ | 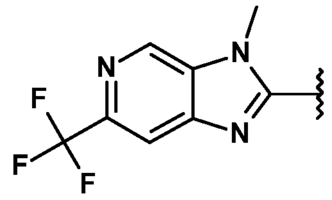 |
| I-24 | $C_2H_5$ | 2 | 8-$CF_3$ | 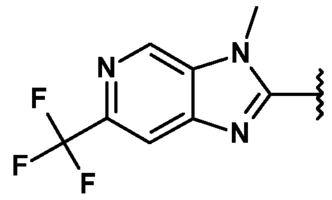 |
| I-25 | $C_2H_5$ | 1 | 8-$CF_3$ | 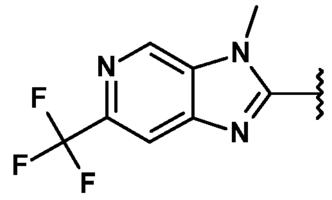 |
| I-26 | $C_2H_5$ | 0 | 5-$CH_3$ | 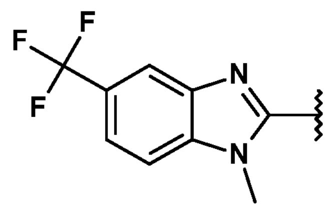 |
| I-27 | $C_2H_5$ | 0 | 5-$CF_3$ | 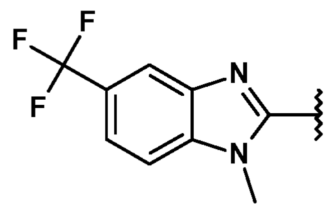 |
| I-28 | $C_2H_5$ | 0 | 6-$CF_3$ | 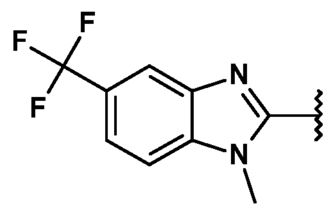 |
| I-29 | $C_2H_5$ | 0 | 6-Cl | 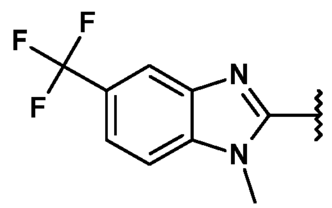 |

(fortgesetzt)

| | $R^1$ | n | $R^2$ | Q |
|---|---|---|---|---|
| I-30 | $C_2H_5$ | 0 | 7-$CF_3$ | 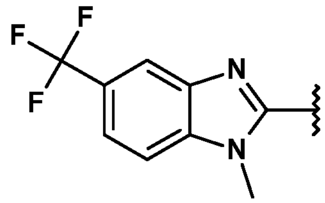 |
| I-31 | $C_2H_5$ | 0 | 5-$SC_2H_5$ | 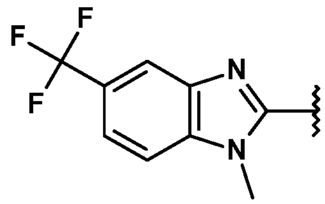 |
| I-32 | $C_2H_5$ | 0 | 7-$SC_2H_5$ | 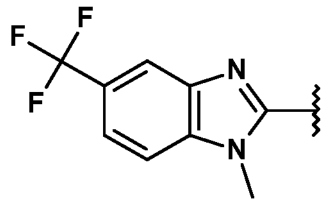 |
| I-33 | $C_2H_5$ | 2 | 7-$CH_3$ | 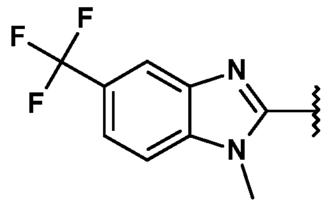 |
| I-34 | $C_2H_5$ | 2 | 7-$CF_3$ | 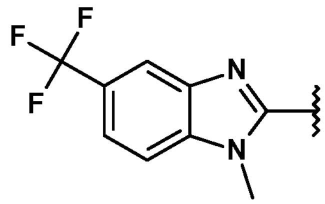 |
| I-35 | $C_2H_5$ | 2 | 6-$CH_3$ | 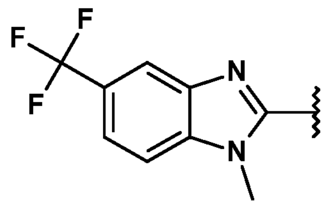 |
| I-36 | $C_2H_5$ | 2 | 6-$CF_3$ | 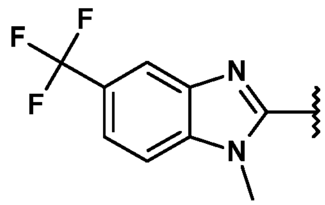 |

(fortgesetzt)

| | $R^1$ | n | $R^2$ | Q |
|---|---|---|---|---|
| I-37 | $C_2H_5$ | 0 | 7-$CH_3$ | 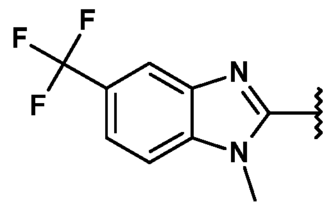 |
| I-38 | $C_2H_5$ | 0 | 6-$CH_3$ | 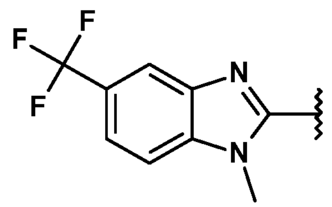 |
| I-39 | $C_2H_5$ | 2 | 8-$SO_2C_2H_5$ | 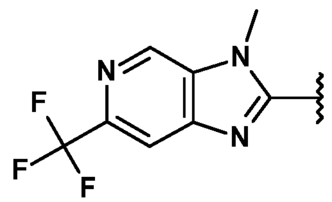 |
| I-40 | $C_2H_5$ | 2 | H | 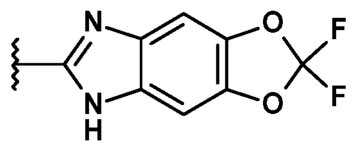 |
| I-41 | $C_2H_5$ | 2 | H | 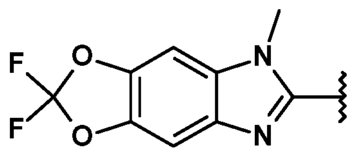 |
| I-42 | $C_2H_5$ | 2 | 6-Cl | 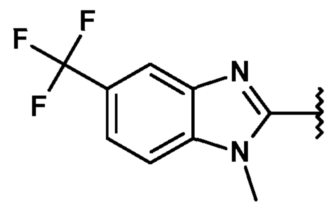 |
| I-45 | $C_2H_5$ | 0 | 6-$CH_3$ | 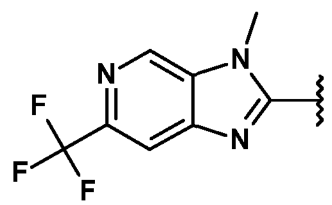 |
| I-46 | $C_2H_5$ | 2 | 5-$CH_3$ | 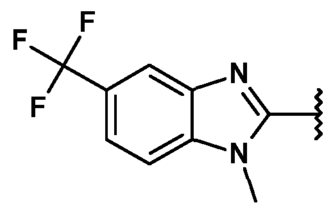 |

(fortgesetzt)

| | $R^1$ | n | $R^2$ | Q |
|---|---|---|---|---|
| I-48 | $C_2H_5$ | 2 | 6-$CH_3$ | 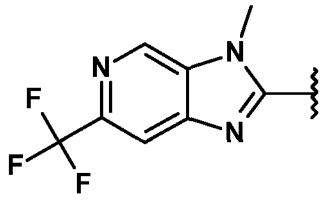 |
| I-54 | $C_2H_5$ | 2 | 6-$OCH_3$ | 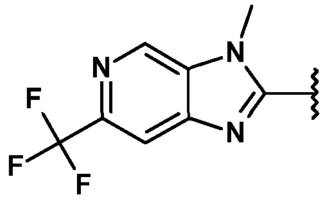 |
| I-55 | $C_2H_5$ | 1 | 6-$OCH_3$ | 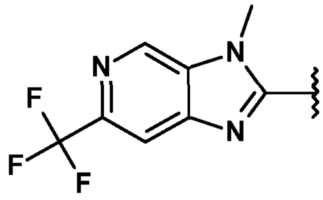 |

**2.** Verbindungen der Formel (XXII)

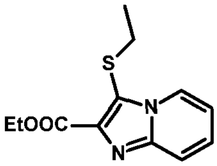

**3.** Verbindungen der Formel (XXIII)

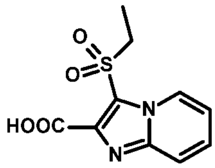

**4.** Verbindungen der Formel (XXIV)

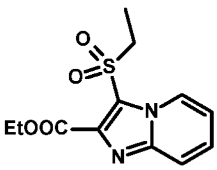

**5.** Verbindungen der Formel (XXVI)

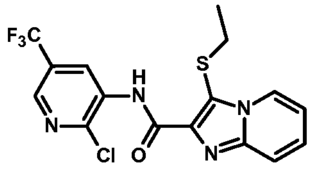

6. Agrochemische Formulierung enthaltend Verbindungen der Formel (I) gemäß Anspruch 1, sowie Streckmittel und/oder oberflächenaktive Substanzen.

7. Agrochemische Formulierung gemäß Anspruch 6 zusätzlich enthaltend einen weiteren agrochemischen Wirkstoff.

8. Verfahren zur Bekämpfung von tierischen Schädlingen **dadurch gekennzeichnet, dass** man eine Verbindung der Formel (I) gemäß Anspruch 1 oder eine agrochemische Formulierung gemäß einem der Ansprüche 6 oder 7 auf die tierischen Schädlinge und/oder ihren Lebensraum einwirken lässt, ausgenommen sind Verfahren zur chirurgischen oder therapeutischen Behandlung des menschlichen oder tierischen Körpers.

9. Verwendung von Verbindungen der Formel (I) gemäß Anspruch 1 oder von agrochemischen Formulierungen gemäß einem der Ansprüche 6 oder 7 zur Bekämpfung von tierischen Schädlingen, ausgenommen ist die Verwendung in Verfahren zur chirurgischen oder therapeutischen Behandlung des menschlichen oder tierischen Körpers.

## Claims

1. Compounds of the formula (I)

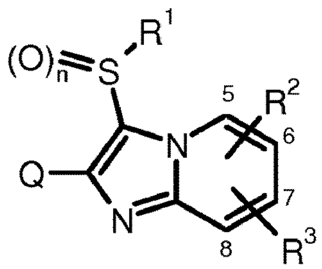

**(I)**

in which $R^3$ represents hydrogen and in which $R^1$, $R^2$, Q and n have the meanings given in the table below and in which the bond from Q to the remainder of the molecule is identified by a wavy line:

| | $R^1$ | n | $R^2$ | Q |
|---|---|---|---|---|
| I-1 | $C_2H_5$ | 2 | H | 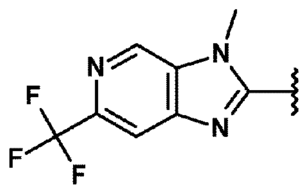 |
| I-2 | $C_2H_5$ | 0 | H | 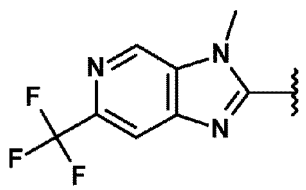 |

(continued)

| | $R^1$ | n | $R^2$ | Q |
|---|---|---|---|---|
| I-7 | $C_2H_5$ | 1 | 6-Cl | 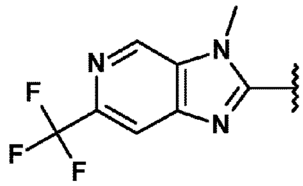 |
| I-8 | $C_2H_5$ | 1 | 6-$CF_3$ | 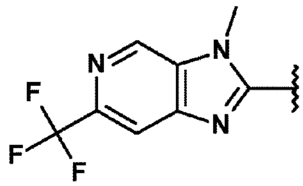 |
| I-9 | $C_2H_5$ | 0 | H | 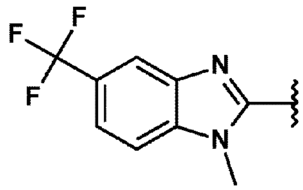 |
| I-10 | $C_2H_5$ | 2 | H | 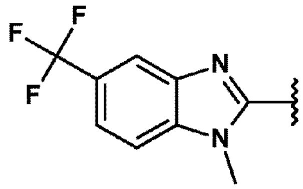 |
| I-11 | $C_2H_5$ | 1 | H | 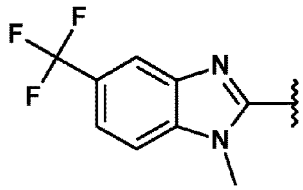 |
| I-13 | $C_2H_5$ | 0 | 8-$CF_3$ | 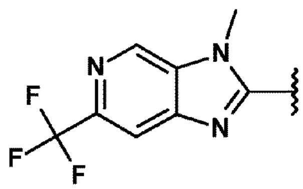 |
| I-14 | $C_2H_5$ | 0 | 8-Cl | 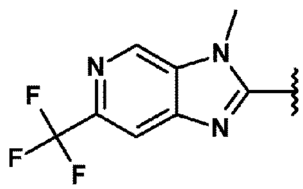 |
| I-15 | $C_2H_5$ | 0 | 8-$SC_2H_5$ | 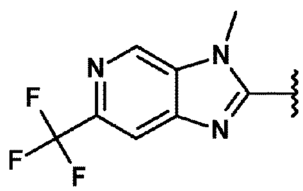 |

(continued)

| | $R^1$ | n | $R^2$ | Q |
|---|---|---|---|---|
| I-18 | $C_2H_5$ | 1 | 7-$CF_3$ | 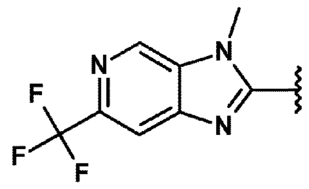 |
| I-19 | $C_2H_5$ | 2 | 8-Cl | 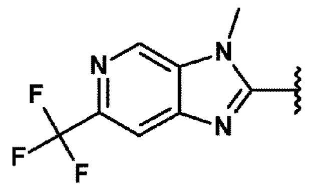 |
| I-20 | $C_2H_5$ | 0 | 5-$CF_3$ | 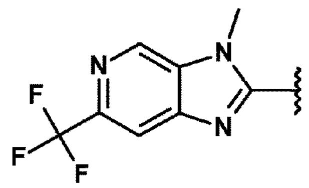 |
| I-21 | $C_2H_5$ | 2 | 5-$CF_3$ | 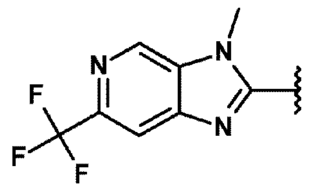 |
| I-22 | $C_2H_5$ | 1 | 5-$CF_3$ | 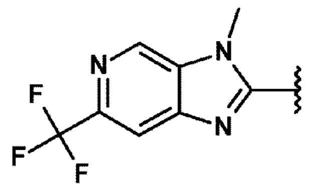 |
| I-24 | $C_2H_5$ | 2 | 8-$CF_3$ | 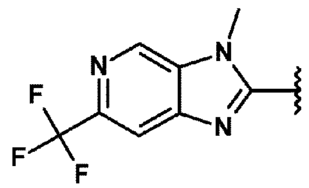 |
| I-25 | $C_2H_5$ | 1 | 8-$CF_3$ | 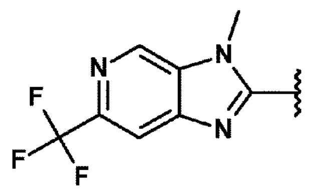 |
| I-26 | $C_2H_5$ | 0 | 5-$CH_3$ | 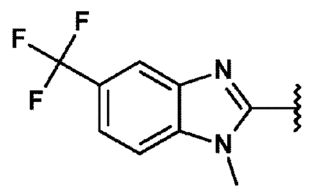 |

(continued)

| | $R^1$ | n | $R^2$ | Q |
|---|---|---|---|---|
| I-27 | $C_2H_5$ | 0 | 5-$CF_3$ | 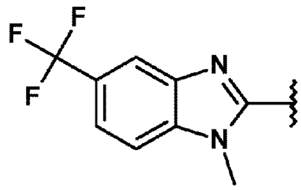 |
| I-28 | $C_2H_5$ | 0 | 6-$CF_3$ | 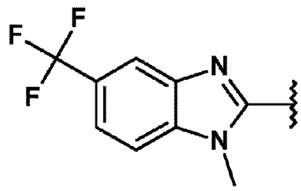 |
| I-29 | $C_2H_5$ | 0 | 6-Cl | 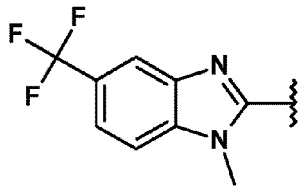 |
| I-30 | $C_2H_5$ | 0 | 7-$CF_3$ | 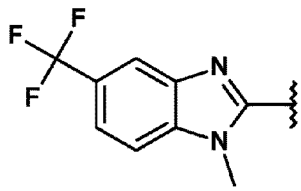 |
| I-31 | $C_2H_5$ | 0 | 5-$SC_2H_5$ | 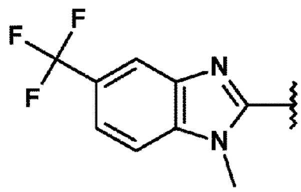 |
| I-32 | $C_2H_5$ | 0 | 7-$SC_2H_5$ | 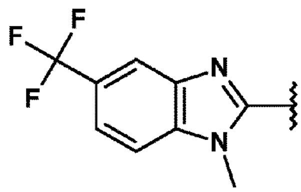 |
| I-33 | $C_2H_5$ | 2 | 7-$CH_3$ | 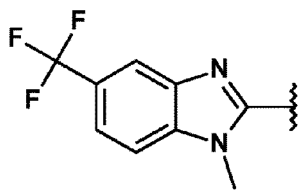 |
| I-34 | $C_2H_5$ | 2 | 7-$CF_3$ | 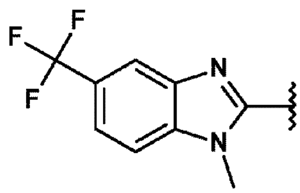 |

(continued)

| | $R^1$ | n | $R^2$ | Q |
|---|---|---|---|---|
| I-35 | $C_2H_5$ | 2 | 6-$CH_3$ | 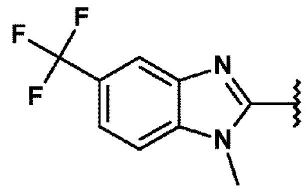 |
| I-36 | $C_2H_5$ | 2 | 6-$CF_3$ | 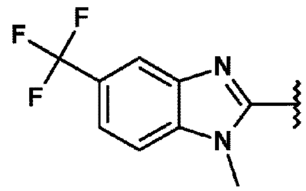 |
| I-37 | $C_2H_5$ | 0 | 7-$CH_3$ | 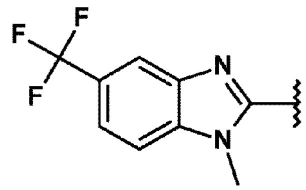 |
| I-38 | $C_2H_5$ | 0 | 6-$CH_3$ | 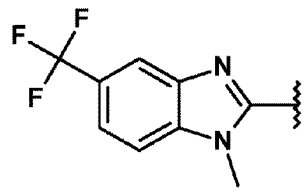 |
| I-39 | $C_2H_5$ | 2 | 8-$SO_2C_2H_5$ | 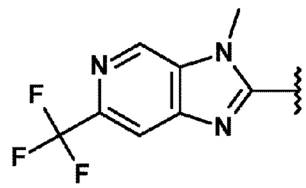 |
| I-40 | $C_2H_5$ | 2 | H | 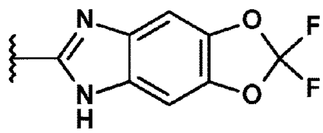 |
| I-41 | $C_2H_5$ | 2 | H | 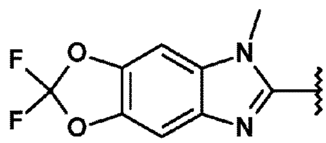 |
| I-42 | $C_2H_5$ | 2 | 6-Cl | 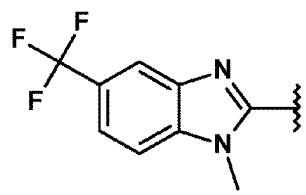 |

(continued)

| | $R^1$ | n | $R^2$ | Q |
|---|---|---|---|---|
| I-45 | $C_2H_5$ | 0 | 6-$CH_3$ | 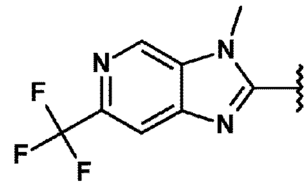 |
| I-46 | $C_2H_5$ | 2 | 5-$CH_3$ | 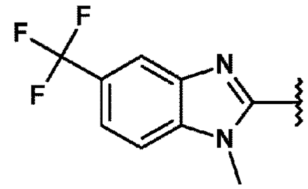 |
| I-48 | $C_2H_5$ | 2 | 6-$CH_3$ | 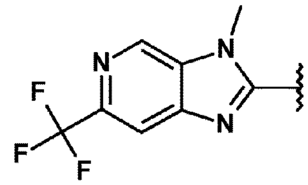 |
| I-54 | $C_2H_5$ | 2 | 6-$OCH_3$ | 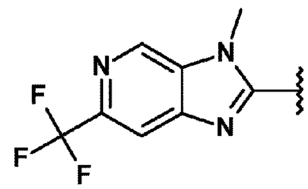 |
| I-55 | $C_2H_5$ | 1 | 6-$OCH_3$ | 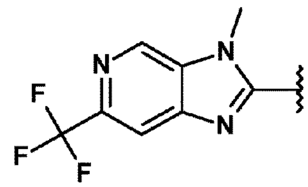 |

**2.** Compounds of the formula (XXII)

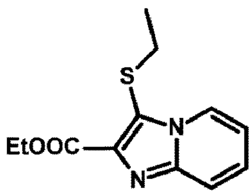

**3.** Compounds of the formula (XXIII)

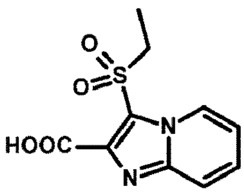

4. Compounds of the formula (XXIV)

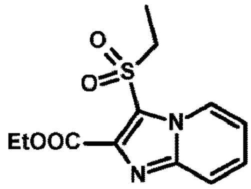

5. Compounds of the formula (XXVI)

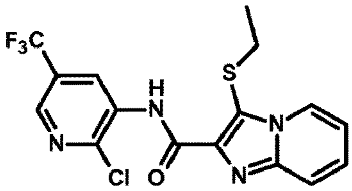

6. Agrochemical formulation, comprising compounds of the formula (I) according to Claim 1 and also extenders and/or surfactants.

7. Agrochemical formulation according to Claim 6, additionally comprising a further agrochemically active compound.

8. Method for controlling animal pests, **characterized in that** a compound of the formula (I) according to Claim 1 or an agrochemical formulation according to either of Claims 6 and 7 is allowed to act on the animal pests and/or their habitat, except for methods for the surgical or therapeutic treatment of the human or animal body.

9. Use of compounds of the formula (I) according to Claim 1 or of agrochemical formulations according to either of Claims 6 and 7 for controlling animal pests, except for the use in methods for the surgical or therapeutic treatment of the human or animal body.

## Revendications

1. Composés de formule (I)

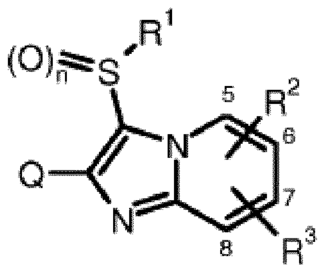

**(I)**

$R^3$ représentant hydrogène et $R^1$, $R^2$, Q et n possédant les significations données dans le tableau suivant et la liaison de Q au reste de la molécule étant indiquée par une ligne ondulée

| | $R^1$ | n | $R^2$ | Q |
|---|---|---|---|---|
| I-1 | $C_2H_5$ | 2 | H | 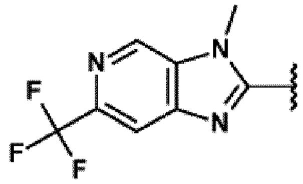 |
| I-2 | $C_2H_5$ | 0 | H | 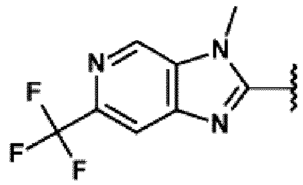 |
| I-7 | $C_2H_5$ | 1 | 6-Cl | 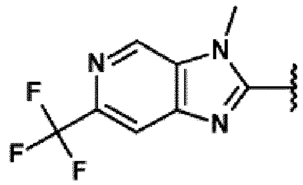 |
| I-8 | $C_2H_5$ | 1 | 6-$CF_3$ | 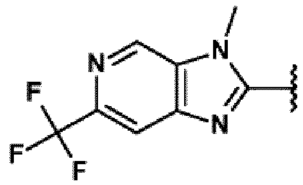 |
| I-9 | $C_2H_5$ | 0 | H | 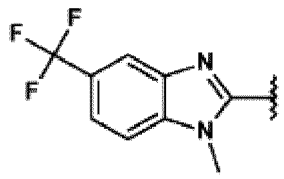 |
| I-10 | $C_2H_5$ | 2 | H | 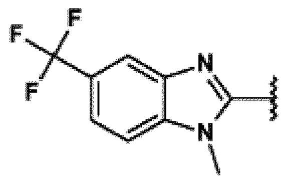 |
| I-11 | $C_2H_5$ | 1 | H | 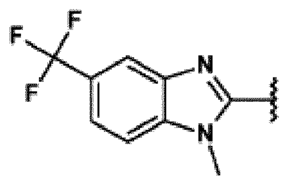 |
| I-13 | $C_2H_5$ | 0 | 8-$CF_3$ | 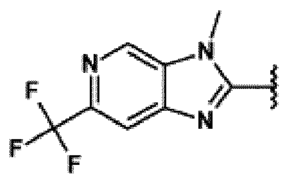 |

(suite)

| | $R^1$ | n | $R^2$ | Q |
|---|---|---|---|---|
| I-14 | $C_2H_5$ | 0 | 8-Cl | 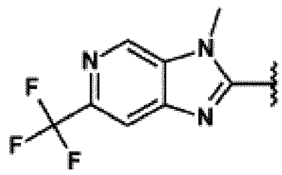 |
| I-15 | $C_2H_5$ | 0 | 8-$SC_2H_5$ | 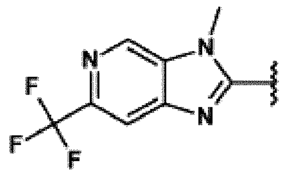 |
| I-18 | $C_2H_5$ | 1 | 7-$CF_3$ | 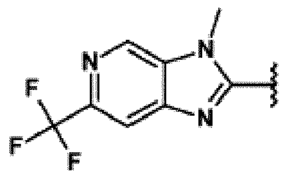 |
| I-19 | $C_2H_5$ | 2 | 8-Cl | 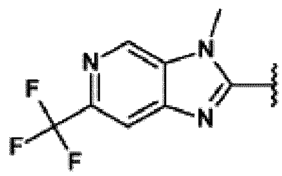 |
| I-20 | $C_2H_5$ | 0 | 5-$CF_3$ | 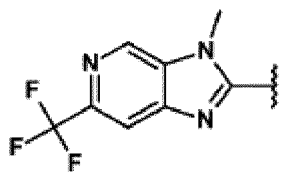 |
| I-21 | $C_2H_5$ | 2 | 5-$CF_3$ | 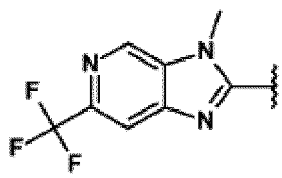 |
| I-22 | $C_2H_5$ | 1 | 5-$CF_3$ | 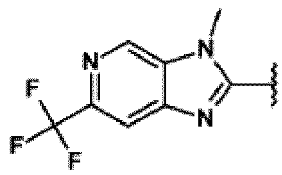 |
| I-24 | $C_2H_5$ | 2 | 8-$CF_3$ | 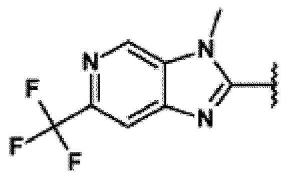 |

(suite)

| | $R^1$ | n | $R^2$ | Q |
|---|---|---|---|---|
| I-25 | $C_2H_5$ | 1 | 8-$CF_3$ | 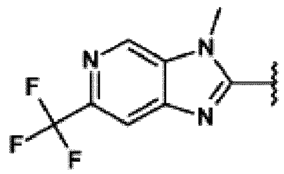 |
| I-26 | $C_2H_5$ | 0 | 5-$CH_3$ | 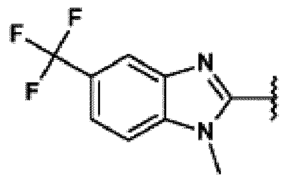 |
| I-27 | $C_2H_5$ | 0 | 5-$CF_3$ | 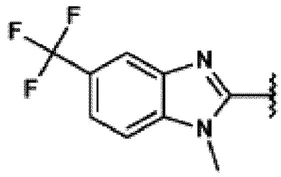 |
| I-28 | $C_2H_5$ | 0 | 6-$CF_3$ | 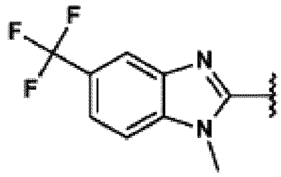 |
| I-29 | $C_2H_5$ | 0 | 6-Cl | 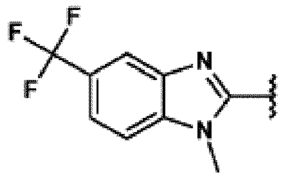 |
| I-30 | $C_2H_5$ | 0 | 7-$CF_3$ | 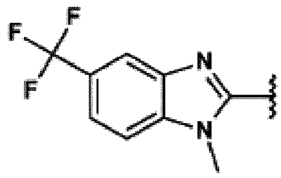 |
| I-31 | $C_2H_5$ | 0 | 5-$SC_2H_5$ | 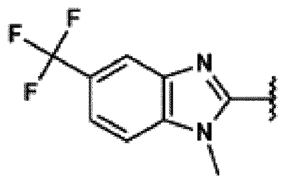 |

(suite)

| | $R^1$ | n | $R^2$ | Q |
|---|---|---|---|---|
| I-32 | $C_2H_5$ | 0 | 7-$SC_2H_5$ | 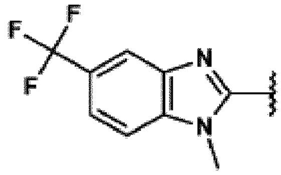 |
| I-33 | $C_2H_5$ | 2 | 7-$CH_3$ | 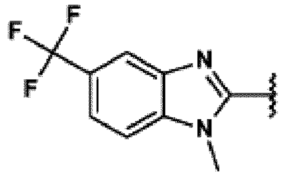 |
| I-34 | $C_2H_5$ | 2 | 7-$CF_3$ | 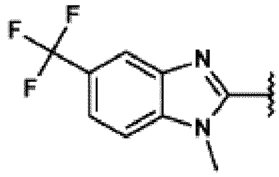 |
| I-35 | $C_2H_5$ | 2 | 6-$CH_3$ | 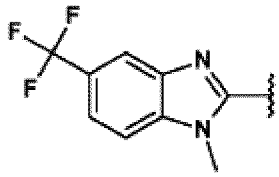 |
| I-36 | $C_2H_5$ | 2 | 6-$CF_3$ | 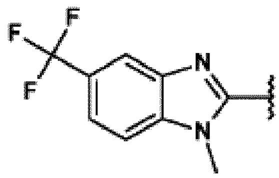 |
| I-37 | $C_2H_5$ | 0 | 7-$CH_3$ | 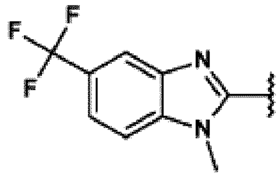 |
| I-38 | $C_2H_5$ | 0 | 6-$CH_3$ | 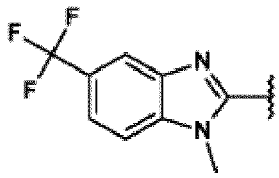 |
| I-39 | $C_2H_5$ | 2 | 8-$SO_2C_2H_5$ | 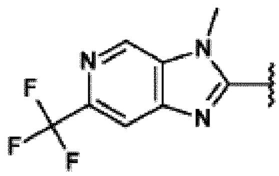 |

(suite)

| | $R^1$ | n | $R^2$ | Q |
|---|---|---|---|---|
| I-40 | $C_2H_5$ | 2 | H | 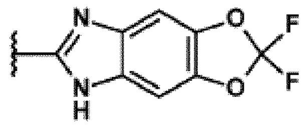 |
| I-41 | $C_2H_5$ | 2 | H | 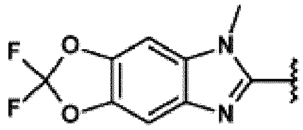 |
| I-42 | $C_2H_5$ | 2 | 6-Cl | 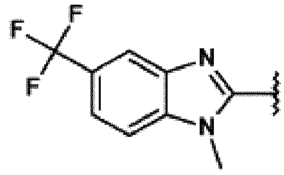 |
| I-45 | $C_2H_5$ | 0 | 6-$CH_3$ | 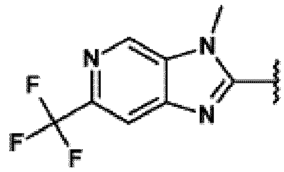 |
| I-46 | $C_2H_5$ | 2 | 5-$CH_3$ | 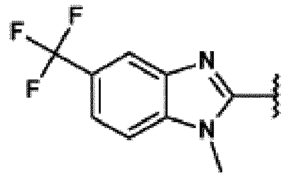 |
| I-48 | $C_2H_5$ | 2 | 6-$CH_3$ | 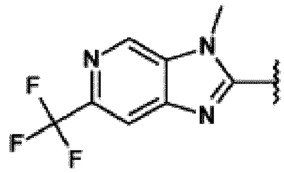 |
| I-54 | $C_2H_5$ | 2 | 6-$OCH_3$ | 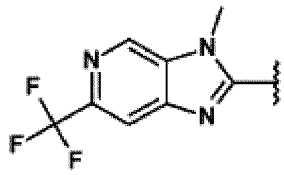 |
| I-55 | $C_2H_5$ | 1 | 6-$OCH_3$ | 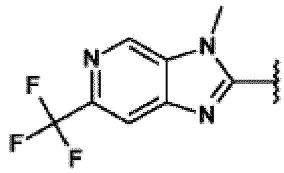 |

**2.** Composés de formule (XXII)

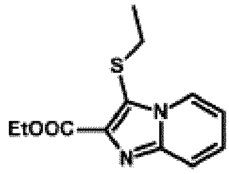

.

3. Composés de formule (XXIII)

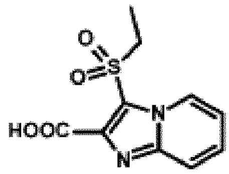

.

4. Composés de formule (XXIV)

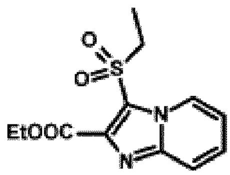

.

5. Composés de formule (XXVI)

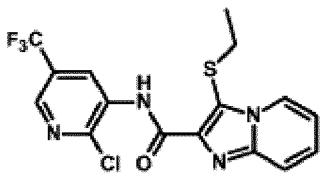

.

6. Formulation agrochimique contenant des composés de formule (I) selon la revendication 1, ainsi que des diluants et/ou des substances tensioactives.

7. Formulation agrochimique selon la revendication 6 contenant de plus un principe actif agrochimique supplémentaire.

8. Procédé pour la lutte contre des parasites animaux **caractérisé en ce qu'**on laisse agir un composé de formule (I) selon la revendication 1 ou une formulation agrochimique selon l'une quelconque des revendications 6 et 7 sur les parasites animaux et/ou sur leur lieu de vie, des procédés pour le traitement chirurgical ou thérapeutique du corps humain ou animal étant exclus.

9. Utilisation de composés de formule (I) selon la revendication 1 ou de formulations agrochimiques selon l'une quelconque des revendications 6 et 7 pour la lutte contre des parasites animaux, l'utilisation dans des procédés pour le traitement chirurgical ou thérapeutique du corps humain ou animal étant exclue.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010125985 A
- WO 2012074135 A
- WO 2012086848 A
- WO 2013018928 A
- WO 2013191113 A
- WO 2014142292 A
- WO 2014148451 A
- WO 2015000715 A
- EP 15191440
- EP 15197267
- EP 16152384
- WO 2016129684 A
- WO 2017061497 A
- WO 2009131237 A
- WO 2011043404 A
- WO 2011040629 A
- US 5576335 A
- US 200369257 B
- WO 200665703 A
- WO 201141713 A
- US 200625633 B
- US 2006111591 A
- US 2820062 A
- US 2009203705 A
- US 2012258951 A
- WO 20133298 A
- WO 201175643 A
- EP 2671582 A
- US 2012302573 A
- WO 200566177 A
- WO 201288411 A
- WO 2012168733 A
- WO 2014187762 A
- US 5374646 A
- WO 2011073149 A
- WO 200851493 A
- WO 2010091310 A
- WO 201266061 A
- WO 2013099041 A
- WO 2005100353 A
- WO 201343518 A
- EP 2168965 A
- US 2003069257 A
- US 20120319050 A
- WO 2011107998 A
- WO 201091310 A
- WO 2011073316 A
- WO 2013018371 A
- WO 2009023179 A
- WO 2011041713 A
- EP 0120598 A1
- EP 0052016 A1
- WO 9711075 A
- WO 2006043635 A
- WO 2003106457 A
- WO 2006003494 A
- WO 2009049851 A
- WO 2004099160 A
- WO 2003076415 A
- WO 2005085216 A
- CN 102057925
- WO 2009080250 A
- WO 2012029672 A
- WO 2009099929 A
- CN 101337940
- WO 2008134969 A
- CN 102060818
- WO 2013144213 A
- WO 2012000896 A
- WO 2010051926 A


### In der Beschreibung aufgeführte Nicht-Patentliteratur


- *Synthesis,* vol. 47 (05), 659-671
- *Chemical Communications,* 2000, vol. 13, 1163-1164
- *Journal of the American Chemical Society,* 1922, vol. 44, 1329
- *J. Med. Chem.,* vol. 31 (1988), 1590-1595
- *J. Med. Chem.,* 1988, vol. 31, 1590-1595
- *Bioorganic and Medicinal Chemistry Letters,* 2003, vol. 13, 1093-1096
- *Bioorganic and Medicinal Chemistry,* 2014, vol. 22 (13), 3515-3526
- *European Journal of Medicinal Chemistry,* 2010, vol. 45, 2214-2222
- *Journal of Medicinal Chemistry,* 2003, vol. 46, 1449-1455

- *Angewandte Chemie Int. Ed.,* 2011, vol. 50, 8944-8947
- *Archiv der Pharmazie,* vol. 335 (1), 27-32
- *Chemical and Pharmaceutical Bulletin,* vol. 39 (11), 2937-2943
- *The Journal of Organic Chemistry,* vol. 41 (22), 3549-3556
- *Tetrahedron,* vol. 69 (38), 8255-8262
- *Chemometrics and Intelligent Laboratory Systems,* vol. 20 (1), 71-84
- *Asian Journal of Organic chemistry,* vol. 3 (5), 609-613
- Chemical Abstracts Service. *Journal of Organic Chemistry,* vol. 46 (5), 1026-1030
- Pesticide Specifications, Manual on development and use of FAO and WHO specifications for pesticides, FAO Plant Production and Protection Papers - 173, prepared by the FAO/WHO Joint Meeting on Pesticide Specifications. Crop Life International, 2004
- **BAUR et al.** *Pesticide Science,* 1997, vol. 51, 131-152
- The Pesticide Manual. British Crop Protection Council, 2012
- *CHEMICAL ABSTRACTS,* 1204776-60-2
- Pesticide Manual
- *CHEMICAL ABSTRACTS,* 129531-12-0
- *CHEMICAL ABSTRACTS,* 71526-07-3
- *CHEMICAL ABSTRACTS,* 52836-31-4
- **R. WEGLER.** Chemie der Pflanzenschutz- und Schädlingsbekämpfungsmittel. Springer Verlag, 1970, vol. 2, 401-412